# EUROPEAN PATENT APPLICATION

(11) **EP 2 452 696 A1**
(43) Date of publication of application: **16.05.2012**
(21) Application number: 09847044.6
(22) Date of filing: 10.07.2009
(51) Int. Cl.: A61L 2/26, A61L 2/18, A61L 12/12

(54) **CASE FOR STERILIZING CONTACT LENSES**

(71) Applicant: Menicon Co., Ltd., Nagoya-shi Aichi 460-0006 (JP)
(72) Inventor: MORI, Osamu, Kasugai-shi Aichi 487-0032 (JP); TOYOHARA, Megumi, Kasugai-shi Aichi 487-0032 (JP)
(74) Representative: Forsythe, Dominic
(86) International application number: PCT/JP2009/003242
(87) International publication number: WO 2011/004439

(57) **Abstract**

A novel case for sterilizing contact lenses (16) wherein a contact lens sterilization process using a hydrogen peroxide solution (44) can be carried out easily and rapidly while effectively preventing both damage to the lenses and deposition of a heavy metal from a metal catalyst (14) on the lenses. The contact lenses are horizontally supported in the hydrogen peroxide solution (44) on a bottom surface of a lens containing portion (26) which opens upward, and a contact preventing member (32) which prevents the contact lenses from coming into contact with the metal catalyst (14) is provided between the metal catalyst (14) and the lens containing portion (26).

## Description

### TECHNICAL FIELD

The present invention relates to a case for sterilizing contact lenses used for sterilizing contact lenses using a hydrogen peroxide solution.

### BACKGROUND ART

When using contact lenses, it is necessary to regularly subject the contact lenses to a sterilization process. This is required for preventing eye infections or eye disorders caused by microorganisms such as bacteria or fungi adhered to the contact lens and maintaining safe and comfortable wear. In recent years, as disclosed in Japan Patent Office, Standard Technologies, "Glasses", page 323 (Non-Patent Document 1) and Japanese Unexamined Patent Publication No. JP-A-2001-242428 (Patent Document 1) or the like, a single-liquid-type cleaning and storage solution to which surfactant and sterilizable preservative are added is frequently used for carrying out sterilization of the contact lens concomitantly. However, for users who have an allergy to sterilizable preservative etc. contained in the single-liquid-type cleaning and storage solution, or users who desire more reliable sterilization effect or the like, a sterilization method of immersing contact lenses in a hydrogen peroxide solution is still utilized. In this method, in consideration of simplicity for users in particular, a hydrogen peroxide disinfection system by using a single liquid instead of using multiple liquids is prevailing, which utilizes sterilization power of the hydrogen peroxide solution. Specifically, the sterilization process is performed by the user himself, who immerses the contact lenses in an aqueous solution of hydrogen peroxide for over several hours.

While a hydrogen peroxide solution is non-toxic after decomposition into water and oxygen, if undecomposed residue thereof exists, it may irritates the eye and cause a problem. To cope with this, it has been proposed to accelerate and adjust decomposition reaction of hydrogen peroxide by using a catalyst made of platinum or the like, so that the hydrogen peroxide will be completely decomposed and neutralized before the termination of the sterilization process of the contact lens. See Japanese Patent No. 2660453 (Patent Document 2), for example.

However, the conventional cases for sterilizing contact lenses which utilizes hydrogen peroxide solution is very hard to use. Especially in recent years, even improvement of usability or the like of this type of cases has not been examined, since the majority of users employ sterilization process utilizing the above-mentioned single-liquid-type cleaning and storage solution. Specifically, as disclosed in Patent Document 2 mentioned earlier, the conventional cases for sterilizing contact lenses which utilizes hydrogen peroxide solution includes a case main unit of deep cylindrical shape that opens upward and a lid that is screw-fastened onto the opening of the case main unit, wherein the lid is furnished with a lens retaining portion and a catalyst retaining portion that jut out into the case main unit. With this case main unit of deep cylindrical shape, the user has to place the left/right pair of contact lens in the lens retaining portion of special bucket shape with the optical axis of the contact lens oriented in approximately horizontal direction and the contact lenses standing (vertically oriented). This will not only make it very cumbersome to place and take out the contact lenses but also pose a risk of breakage or the like of contact lenses by being pinched by the bucket while being placed therein. Moreover, since the case main unit is deep, especially from the latter half of decomposition and neutralization when generation of oxygen gas reduces, stirring of the hydrogen peroxide solution contained therein may become inefficient. A resultant risk is that decomposition and neutralization of the hydrogen peroxide solution may partially progress or the like and may cause unevenness of the sterilization process and the neutralization process. Furthermore, since the case main unit is of deep cylindrical shape and tall, even if placed on a table or the like, it easily falls over when touched by fingers, resulting in a problem that the hydrogen peroxide solution is likely to spill out.

Meanwhile, it would be conceivable to utilize a contact lens storage container as disclosed in Japanese Unexamined Patent Publication No. JP-A-2000-189224 (Patent Document 3) or in Japanese Unexamined Patent Publication No. JP-A-6-205706 (Patent Document 4) for sterilization utilizing a hydrogen peroxide solution. This container is oriented horizontally and furnished with a pair of shallow lens containing portions that open upward and contain the contact lens in a horizontal manner, with its optical axis oriented in approximately vertical direction. However, the Patent Documents 3 and 4 never disclose specifically where and in what structure a catalyst for neutralizing hydrogen peroxide solution should be disposed in such a contact lens storage container oriented horizontally. Besides, these documents have never even examined an effective assembly structure of such catalysts.

Another example is a container for sterilizing contact lenses which utilizes a hydrogen peroxide solution as proposed in Japanese Domestic Publication of International Patent Application No. JP-A-2002-526203 (Patent Document 5), wherein a contact lens storage container oriented horizontally as described above has shallow lens containing portions whose bottom surface and peripheral wall inner surface are simply coated with a platinum layer. However, with such a simple structure in which the entire inner surface of the container is coated with a catalyst layer, there is a risk that the contact lens may cover the surface of the catalyst layer and deteriorate the decomposition efficiency of the catalyst. In addition, when the contact lens touches the catalyst, there is a risk that heavy metals may adhere to the contact lens, causing a consequent risk of inducing troubles such as user's allergy, or damaging the lenses.

Japanese Patent No. 3368903 (Patent Document 6) proposes to neutralize the hydrogen peroxide solution by using an enzyme tablet containing catalase instead of using the catalyst. However, the enzyme tablet must be put into the hydrogen peroxide solution at the time of each sterilization process. This operation or handling is cumbersome, and there is a risk that the user may forget to put in the enzyme tablet. Moreover, since there is a problem that components of the enzyme tablet may remain even after neutralization of the hydrogen peroxide solution, this proposal is not necessarily an effective approach.

### BACKGROUND ART DOCUMENTS

### PATENT DOCUMENTS

Patent Document 1: Japanese Unexamined Patent Publication No. JP-A-2001-242428
Patent Document 2: Japanese Patent No. 2660453
Patent Document 3: Japanese Unexamined Patent Publication No. JP-A-2000-189224
Patent Document 4: Japanese Unexamined Patent Publication No. JP-A-6-205706
Patent Document 5: Japanese Domestic Publication of International Patent Application No. JP-A-2002-526203
Patent Document 6: Japanese Patent No. 3368903

### NON-PATENT DOCUMENT

Non-Patent Document 1: Japan Patent Office, Standard Technologies, General, year 2005, "Glasses", page 323, Title "13-3-1-1 Storage Method in Solution"

### SUMMARY OF THE INVENTION

### PROBLEM THE INVENTION ATTEMPTS TO SOLVE

It is an object of the present invention to provide a case for sterilizing contact lenses with a novel constitution which enables the user to carry out a contact lens sterilization process easily, especially making it possible to place and take out the contact lenses readily and rapidly, as well as to effectively prevent problems caused by a metal catalyst such as deposition of a heavy metal to the lenses, damage to the lenses, or the like.

### MEANS FOR SOLVING THE PROBLEM

A first mode of the present invention provides a case for sterilizing contact lenses including: a pair of lens containing portions each opening upward for immersing and sterilizing a contact lens for a left eye and a contact lens for a right eye within a hydrogen peroxide solution, a bottom surface of each of the lens containing portions being adapted to support the contact lens horizontally in a state where either one of a concave side lens surface and a convex side lens surface faces toward an opening of the lens containing portion; a metal catalyst which catalyzes decomposition reaction of the hydrogen peroxide solution, being provided at a location where the metal catalyst comes into contact with the hydrogen peroxide solution; and a contact preventing member provided between the metal catalyst and the lens containing portion for preventing the contact lens from coming into contact with the metal catalyst.

With the catalyst for contact lens sterilization according to the present mode, the contact lens for a left eye and the contact lens for a right eye is adapted to be contained respectively in each of the pair of lens containing portions in a horizontal state. Accordingly, the contact lens can be directly put in or taken out from the lens containing portion through the opening of the lens containing portion with the case for sterilizing contact lenses placed on the resting face such as a table or the like. In particular, the each lens containing portion is made sufficiently shallow in comparison with the conventional case which is placed vertically. Thus, the operation will be easy since the user is able to directly insert his or her finger through the opening and put in or take out the contact lens, and it will also be easy to verify that the contact lens is present in the lens containing portion.

Moreover, by employing the metal catalyst as the catalyst for decomposition of the hydrogen peroxide solution, unlike the enzyme tablet as disclosed in Patent Document 6, it is not necessary to input the catalyst into the solution each time the process is carried out. Thus, the neutralization reaction will be exhibited with excellent durability, stability, and reliability.

In addition, since the contact preventing member prevents the metal catalyst from coming into direct contact with the contact lens, problems such as deposition of a heavy metal to the lens, damage to the lens, or the like can be avoided as practicably as possible.

A second mode of the present invention provides the case for sterilizing contact lenses according to the first mode wherein the metal catalyst is positioned below the contact lens adapted to be contained in the lens containing portion.

According to the present mode, since the contact lens, which is the target of the sterilization, is disposed above the rising oxygen gas which has been generated around the catalyst, the circulation action will effectively take place in the area where the contact lens is positioned in association with rise of the oxygen gas in the lens containing portion. Besides, it can be expected that the dirt will be removed from the contact lens by contact action of the bubbles of the oxygen gas. Moreover, by providing the containing area of the catalyst below the containing area of the contact lens, it is possible to make the entire size of the case for sterilizing contact lenses in the plane surface compact or small.

A third mode of the present invention provides the case for sterilizing contact lenses according to the first or second mode, further including: a solution containing portion for containing the hydrogen peroxide solution; and a protruding support part projecting from a bottom surface of the solution containing portion, wherein the lens containing portion is formed at a projecting distal end of the protruding support part.

According to the present mode, even if the solution containing portion is deep and has a large capacity for obtaining a sufficient containing portion or the like, the lens containing portion can be made shallow, so that the user is easily able to place and taking out the contact lens as well as to visually observe that the contact lens is present.

A fourth mode of the present invention provides the case for sterilizing contact lenses according to the third mode wherein the protruding support part includes a leg portion projecting from the bottom surface of the solution containing portion and an umbrella-shaped head portion flaring peripherally outward from a projecting distal end of the leg portion, and the lens containing portion is formed at the umbrella-shaped head portion.

A fifth mode of the present invention provides the case for sterilizing contact lenses according to the fourth mode wherein in the solution containing portion, the metal catalyst is positioned below the umbrella-shaped head portion, the contact preventing member comprises the umbrella-shaped head portion of the protruding support part, and the umbrella-shaped head portion limits movement of the metal catalyst within the solution containing portion so as to prevent the metal catalyst from coming into contact with the contact lens adapted to be contained in the lens containing portion of the umbrella-shaped head portion.

According to the present mode, it is desirable that the umbrella-shaped head portion have a porous structure or a net structure, and that the metal catalyst be positioned below the lens containing portion formed in the umbrella-shaped head portion. With this arrangement, it is possible to lead the oxygen gas, which is generated in association with the decomposition reaction of the hydrogen peroxide solution by means of the metal catalyst, toward the lens containing portion, thereby inducing stir or convection current of the hydrogen peroxide solution.

A sixth mode of the present invention provides the case for sterilizing contact lenses according to any of the first through fifth modes wherein, as the contact preventing member, a passage constricting member is provided between areas where the contact lens and the metal catalyst are positioned in order to prevent movement of both the contact lens and the metal catalyst while permitting flow of the hydrogen peroxide solution.

According to the present mode, it is not necessary for the metal catalyst to be secured, bonded, or the like to the case, so that a wide choice for the metal catalyst as well as a simple fabrication or structure thereof can be obtained. As specific examples of the passage constricting member, a net, a fence, a porous material, a constricting passage or the like could be employed.

A seventh mode of the present invention provides the case for sterilizing contact lenses according to any of the first through sixth modes, further including a temporary lens platform for temporarily retaining the contact lens, the temporary lens platform being provided at a surface away from the solution containing portion which is adapted to contain the hydrogen peroxide solution.

Specifically, with the case for sterilizing contact lenses utilizing the hydrogen peroxide solution, if the user removes the contact lens for one eye to immerse it in the solution and then intends to remove the contact lens for the other eye, there is a risk that the hydrogen peroxide solution adhered to the user's finger during immersing the contact lens for one eye may touch the other eye. According to this mode, it is readily possible to place the first-removed contact lens for one eye on the temporary lens platform and then remove the contact lens for the other eye, and afterwards immerse the both contact lenses in the hydrogen peroxide solution. By so doing, problems that the hydrogen peroxide solution may touch the user's eye during the contact lens sterilization process will be effectively prevented.

An eighth mode of the present invention provides the case for sterilizing contact lenses according to any of the first through seventh modes wherein: the solution containing portion which is adapted to contain the hydrogen peroxide solution includes the lens containing portion; a lid is provided for covering the solution containing portion; and a gas discharge passage is provided for discharging an oxygen gas, which is generated in association with a decomposition of the hydrogen peroxide solution, from the solution containing portion with the solution containing portion covered by the lid.

The gas discharge passage according to the present mode may be formed by providing a through hole to the lid or the like, for example. Alternatively, it would also be acceptable to utilize a gap between the lid and the case main unit onto which the lid is adapted to be superposed.

A ninth mode of the present invention provides the case for sterilizing contact lenses according to the eighth mode wherein the lid is provided in a bendable manner to a case main unit which forms the solution containing portion, and the solution containing portion is adapted to be covered by means of the lid being bent with respect to the case main unit so as to be superposed against an upper face of the case main unit onto which the solution containing portion opens.

In order to make the case main unit and the lid bendable in the present mode, for example, the case main unit and the lid formed as separate elements may be linked in a bendable manner by a flexible tape, a hinge member, or the like. Alternatively, as will be described later in the tenth mode, an easily bendable part may be provided between the integrally-formed case main unit and lid.

A tenth mode of the present invention provides the case for sterilizing contact lenses according to the ninth mode wherein the case main unit and the lid is integrally molded from a thin-walled resin material, and the lid is bendable by being bent at a linking portion of the case main unit and the lid.

The present mode will be advantageously realized by integrally forming the case main unit and the lid with a film material made of a synthetic resin for example, and linking them at the linear section of each outer peripheral edge thereof in a bendable manner.

An eleventh mode of the present invention provides the case for sterilizing contact lenses according to any of the first through tenth modes, further including a catalyst containing portion which contains the metal catalyst, wherein the pair of lens containing portions and the catalyst containing portion are respectively formed as recesses that open upward, and mutual communication passages are formed for mutually connecting the recesses so as to allow the hydrogen peroxide solution to flow among the recesses.

According to the present mode, the left/right pair of the lens containing portions and the catalyst containing portion are formed so as to open onto the different portions from one another. This makes it possible to obtain a sufficient capacity for each of the containing portions while minimizing the maximum height dimension of the case for sterilizing contact lenses. Besides, this arrangement makes it easy not only to place and take out the contact lenses with respect to the pair of lens containing portions but also to place and take out the metal catalyst with respect to the catalyst containing portion or the like. Furthermore, it is possible to directly view and check the condition of the metal catalyst immersed within the hydrogen peroxide solution through the opening of the catalyst containing portion.

A twelfth mode of the present invention provides the case for sterilizing contact lenses according to the eleventh mode wherein each of the pair of lens containing portions has an aperture of the mutual communication passage, and the aperture has a length equal to 1/5 or more of a circumference of the lens containing portion.

According to the present mode, among the catalyst containing portion and the pair of lens containing portions, the circulation efficiency of the hydrogen peroxide solution contained therein will be improved and maintained. By so doing, the neutralization reaction of the hydrogen peroxide solution that actively progresses in the catalyst containing portion will be efficiently exerted as far as to the pair of lens containing portions, thereby advantageously uniforming the entire concentration (the progress of the neutralization reaction) of the solution.

A thirteenth mode of the present invention provides the case for sterilizing contact lenses according to the eleventh or twelfth mode wherein the mutual communication passages interconnect the recesses so as to form an annular circulation passage.

According to the present mode, more improved circulation efficiency of the hydrogen peroxide solution can be obtained among the catalyst containing portion and the pair of lens containing portions. The catalyst containing portion may be constituted, for example, in the way taught by the fourteenth mode to be described later. By so doing, one catalyst containing portion is able to even more efficiently communicate with the pair of lens containing portions.

A fourteenth mode of the present invention provides the case for sterilizing contact lenses according to the thirteenth mode wherein: the catalyst containing portion is positioned between the pair of lens containing portions; a pair of adjacent communication passages each connecting with one of the lens containing portions are formed on a rim of an opening of the catalyst containing portion at positions opposed to each other so that the pair of lens containing portions are interconnected via the catalyst containing portion; and a parallel communication passage is formed so as to extend parallel to a direction of array of the pair of lens containing portions and the catalyst containing portion, and lengthwise opposite ends of the parallel communication passage each connect with one of the lens containing portions while a lengthwise middle portion of the parallel communication passage connects with the catalyst containing portion so that the circulation passage includes the pair of adjacent communication passages and the parallel communication passage.

A fifteenth mode of the present invention provides the case for sterilizing contact lenses according to any of the eleventh through fourteenth modes, further including a common recess that surrounds a periphery of openings of the pair of lens containing portions and the catalyst containing portion, wherein the pair of lens containing portions and the catalyst containing portion open onto the common recess.

According to the present mode, for example, if the user's finger is inserted into the lens containing portion during placing and taking out of the contact lens with respect to the lens containing portion, the hydrogen peroxide solution pushed out by the user's finger will rapidly flow via the common recess to the other lens containing portion or to the catalyst containing portion etc. Therefore, the rise of the surface of the hydrogen peroxide solution in the lens containing portion will be minimized, thereby effectively preventing the hydrogen peroxide solution from spilling out of the case.

A sixteenth mode of the present invention provides the case for sterilizing contact lenses according to any of the eleventh through fifteenth modes wherein the lid that covers the opening of the pair of lens containing portions has on an inside face thereof inward convex portions corresponding with the lens containing portions, and by means of the lid being mounted onto the opening of the lens containing portions, the inward convex portions are adapted to be inserted into the hydrogen peroxide solution contained in the lens containing portions.

According to the present mode, by placing the contact lenses in the pair of lens containing portions and then attaching the lid, the inward convex portions of the lid will push the hydrogen peroxide solution out of the pair of lens containing portions. This will raise the surface level of the hydrogen peroxide solution, so that the solution surface level of the catalyst containing portion will rise as well, thereby making it possible to immerse the catalyst in more reliable manner.

A seventeenth mode of the present invention provides the case for sterilizing contact lenses according to the sixteenth mode wherein by means of the lid being mounted onto the opening of the lens containing portions, the inward convex portions are adapted to push the contact lenses into the hydrogen peroxide solution contained in the lens containing portions.

An eighteenth mode of the present invention provides the case for sterilizing contact lenses according to any of the first through seventeenth modes wherein the metal catalyst is at least one of metals and metal oxides thereof selected from the group consisting of platinum, silver, palladium, copper, manganese, cobalt, and aluminum.

By employing the metal catalyst as described above, a good catalysis will be stably exhibited for multiple times of use. Whereas the entire catalyst may be formed of the material of the metal catalyst, it would also be acceptable for the catalyst to be formed of a composite material, as will be taught in the twentieth mode described later.

A nineteenth mode of the present invention provides the case for sterilizing contact lenses according to any of the first through eighteenth modes wherein the metal catalyst has a surface area of between 3 and 30 cm² per 10 ml of the hydrogen peroxide solution.

If the surface area of the catalyst is smaller than 3 cm²/10 ml, there is a risk that the catalysis may not sufficiently exhibit on neutralization reaction of the hydrogen peroxide solution, resulting in requiring a long time for the neutralization process and hence for the contact lens sterilization process. On the other hand, if the surface area of the catalyst is greater than 30 cm²/10 ml, not only may the production cost of the catalyst be high, but also there is a risk that the neutralization reaction may progress so fast that the contact lens sterilization process may be insufficient.

A twentieth mode of the present invention provides the case for sterilizing contact lenses according to any of the first through nineteenth modes wherein the metal catalyst is made by adhering a metal coat which catalyzes decomposition reaction of the hydrogen peroxide solution to a surface of a base material.

As the base material, it is possible to employ a suitable material such as a synthetic resin material, a metal, a glass, a ceramic, a rubber, or the like, for example. A metal coat can be formed on a part or the entirety of the base material surface. By so doing, according to the present mode, a large surface area will be established while using a limited amount of the metal catalyst, so that adjustments such as improving catalysis will be possible. In addition, there is a greater degree of freedom in designing the shape of the catalyst depending on the place where the catalyst is contained or the like.

A twenty-first mode of the present invention provides the case for sterilizing contact lenses according to any of the first through twentieth modes wherein: the metal catalyst is disposed in the catalyst containing portion and adapted to be immersed within the hydrogen peroxide solution; during decomposition reaction of the hydrogen peroxide solution, the metal catalyst undergoes displacement by exertion of buoyancy on the basis of oxygen bubbles being generated; and after decomposition reaction of the hydrogen peroxide solution, the metal catalyst is held in non-displacement state due to dissipation of the buoyancy on the basis of the oxygen bubbles while being displaceable within the catalyst containing portion.

According to the present mode, at least in the state where a normal catalysis is exhibited, the catalyst undergoes displacement within the solution. Therefore, by visually observing the displacement of the catalyst in addition to the oxygen bubbles generated during decomposition of the hydrogen peroxide solution, the user of the contact lens can more easily appreciate that both the desired sterilization and the expected neutralization are being performed. Accordingly, in comparison with only observing the oxygen bubbles, the user of the contact lens is able to more clearly confirm that the expected sterilization process is being performed, thereby affording sense of security.

In addition, owing to the displacement of the catalyst induced by the oxygen bubbles, the hydrogen peroxide solution can be stirred on the basis of the displacement of the catalyst within the hydrogen peroxide solution and diffusion of the oxygen bubbles. Consequently, the uniform hydrogen peroxide solution will be obtained, thereby attaining both stable sterilization process of the contact lens and stable neutralization process expected with respect to the hydrogen peroxide solution.

In the present embodiment in particular, the displacement of the catalyst can be realized by utilizing buoyancy of the oxygen bubbles. Thus, neither special energy nor large-scale apparatus or the like is necessary. Moreover, the oxygen bubbles, which serve as the motive power of the displacement of the catalyst, are generated in association with decomposition of the hydrogen peroxide solution by means of the catalyst. This will be an indicator that the expected decomposition process of the hydrogen peroxide solution by means of the catalyst has also been normally started at the start of the contact lens sterilization process by means of the hydrogen peroxide solution.

Furthermore, the displacement of the catalyst within the hydrogen peroxide solution during the sterilization process can attract the interest of the user. This will motivate the user to voluntarily carry out the sterilization process and check the status thereof. Therefore, it can be expected that the sterilization process of the contact lens entrusted to the user will be regularly performed in more reliable and stable manner.

A twenty-second mode of the present invention provides the case for sterilizing contact lenses according to the twenty-first mode wherein the displacement of the metal catalyst during decomposition reaction of the hydrogen peroxide solution is rotation; and the non-displacement state of the metal catalyst after decomposition reaction of the hydrogen peroxide solution is stop of rotation.

A twenty-third mode of the present invention provides the case for sterilizing contact lenses according to the twenty-first or twenty-second mode wherein the displacement of the metal catalyst during decomposition reaction of the hydrogen peroxide solution is emergence; and the non-displacement state of the metal catalyst after decomposition reaction of the hydrogen peroxide solution is submergence.

According to the above-mentioned twenty-second or twenty-third mode, the energy of the oxygen bubbles generated during the decomposition reaction of the hydrogen peroxide solution can be efficiently utilized for the displacement of the catalyst. Note that it would also be possible to concomitantly manifest both of the rotation according to the twenty-second mode and the emergence according to the twenty-third mode. It could also be acceptable to employ other displacement mode such as swinging or the like instead of or in addition to the above-mentioned rotation or emergence.

In particular, by employing the rotational displacement according to the twenty-second mode, the catalyst continues to displace as long as the oxygen bubbles are generated by more than the prescribed amount per unit time by means of decomposition reaction of the hydrogen peroxide solution, making it possible to readily confirm whether or not the catalyst undergoes displacement. In order to induce rotational displacement of the catalyst by means of buoyancy of the oxygen bubbles, for example, a motion conversion mechanism of conventionally known such as a water wheel that rotates utilizing gravity or the like may be employed.

Meanwhile, by employing the emerging/submerging displacement according to the twenty-third mode, the displacement of the catalyst can be more easily observed, especially from the lateral side. Note that the catalyst emerges and submerges by such an amount of displacement within the hydrogen peroxide solution as to be visually checked. In preferred practice, in the emerging state, the catalyst is adapted to be partially exposed upward out of the solution surface while in the submerging state, the catalyst is adapted to be in contact with the bottom of the container which contains the hydrogen peroxide solution.

A twenty-fourth mode of the present invention provides the case for sterilizing contact lenses according to any of the twenty-first through twenty-third modes wherein the metal catalyst has on a surface thereof at least one of a concave portion and a convex portion.

According to the present mode, "the concave portion" will trap the oxygen bubbles and increase the surface area of the catalyst, thereby obtaining increased buoyancy. Meanwhile, "the convex portion" will increase the surface area of the catalyst and increase bumps of the oxygen bubbles, thereby obtaining increased buoyancy. By appropriately setting the shape, size, number or the like of the concave portion or the convex portion, it would also be possible to adjust the surface area and hence the catalysis of the catalyst.

A twenty-fifth mode of the present invention provides the case for sterilizing contact lenses according to the twenty-fourth mode wherein the metal catalyst includes on the surface thereof a hollow serving as the concave portion that opens downward with the metal catalyst immersed within the hydrogen peroxide solution, and the metal catalyst further includes a discharge passage for discharging the oxygen bubbles trapped in the hollow in a limiting manner.

According to the present mode, during the decomposition reaction of the hydrogen peroxide solution, the hollow traps and pools the generated oxygen bubbles and is able to exert an ample buoyancy on the catalyst. Concomitantly, after the decomposition reaction of the hydrogen peroxide solution, the discharge passage discharges the oxygen gas stored in the hollow and is able to dissipate or decrease the buoyancy action of the oxygen bubbles. This arrangement will utilize the oxygen bubbles even better, realizing displacement of the catalyst such as emergence/submergence or the like.

In addition, by relatively adjusting the size of the hollow and the discharge efficiency of the discharge passage, even in the state where the decomposition reaction of the hydrogen peroxide solution becomes progressively attenuated, for example, it is possible to reserve the oxygen bubbles within the hollow and continue to exert the effective buoyancy on the catalyst so as to keep the catalyst in a state of displacement. It would also be possible to limit the outflow of the oxygen bubbles through the discharge passage so that, when the neutralization of the hydrogen peroxide solution is completed due to its decomposition reaction, the amount of reserved oxygen bubbles in the hollow decreases to the extent that the effective buoyancy will not be exerted on the catalyst.

As "the discharge passage" according to the present mode, for example, it is possible to employ a tunnel structure that perforates the catalyst so as to extend upward from the upper base part of the hollow that opens downward. Alternatively, a groove structure provided to the outer peripheral wall of the hollow (namely, a groove structure having a slightly small height and permitting the bubbles to overpass so as to readily exit to the outer peripheral side) or the like may also be employed.

A twenty-sixth mode of the present invention provides the case for sterilizing contact lenses according to any of the twenty-first through twenty-fifth modes wherein with respect to the metal catalyst, a ratio M/F between a mass M and a buoyancy F exerted within the hydrogen peroxide solution is more than 1 and does not exceed 2.

According to the present mode, apart from the specific gravity as the material, the buoyancy that exerts on the catalyst per se as the component is established within the range of the present mode. With this arrangement, the displacement of emergence/submergence etc. will be effectively realized by presence or absence of the buoyancy action added by the oxygen bubbles of such extent as to be generated during the decomposition of the hydrogen peroxide solution. Note that the value of the buoyancy F according to the present mode is the buoyancy of the catalyst per se and does not include the buoyancy added by the oxygen bubbles. Besides, the buoyancy F can be adjusted by using multiple types of materials in combination, employing a hollow structure, or the like, in consideration of the specific gravity of the material of the catalyst.

A twenty-seventh mode of the present invention provides the case for sterilizing contact lenses according to any of the twenty-first through twenty-sixth modes, further including a reserving member that permits displacement of the metal catalyst within the catalyst containing portion in emerging and submerging directions while reserving the metal catalyst within the catalyst containing portion during discharge of the hydrogen peroxide solution from the catalyst containing portion.

According to the present mode, when discharging the contained hydrogen peroxide solution after the neutralization or the like, the catalyst is prevented from being washed away. This makes it easy to use the case repeatedly for sterilization processes of multiple times.

A twenty-eighth mode of the present invention provides the case for sterilizing contact lenses according to the twenty-seventh mode wherein the metal catalyst includes a mating portion, and on the basis of mating action of the mating portion, displacement of emergence and submergence of the metal catalyst within the catalyst containing portion is permitted while detachment of the metal catalyst from the catalyst containing portion is prevented so as to provide the reserving member.

As "the mating portion and retaining portion" according to the present mode, it is possible to employ various types of structures that are capable of permitting displacement of the catalyst while preventing detachment of the catalyst from the catalyst containing portion. Such structures include, for example, a support shaft mechanism in which the catalyst is furnished with a support shaft that serves as the mating portion and is retained by the retaining portion rotatably about its center axis, and the catalyst undergoes swinging displacement about the support shaft. Another example is a guide mechanism in which the catalyst is furnished with a guide groove that serves as the retaining portion or the mating portion guided by a guide shaft, and undergoes reciprocating displacement. Yet another example is a shaft support mechanism in which the catalyst is furnished with the mating portion rotatably supported by a rotating shaft that serves as the retaining portion. Any of these structures may be preferably employed.

A twenty-ninth mode of the present invention provides the case for sterilizing contact lenses according to the twenty-eighth mode wherein the case for sterilizing contact lenses as defined in the fourteenth mode is employed, and the mating portion provided to the metal catalyst projects out from the catalyst containing portion so as to be mated with the parallel communication passage.

According to the present mode, by utilizing the parallel communication passage well, the reserving member for reserving the metal catalyst within the catalyst containing portion is effectively realized. In particular, the support shaft is rotatably detained within the parallel communication passage while the metal catalyst is formed so as to extend orthogonally from the rotation center axis of the support shaft. With this arrangement, it is possible to establish a large amount of displacement of the metal catalyst induced in association with rotation action of the support shaft without necessitating displacement of the support shaft.

### EFFECT OF THE INVENTION

According to the present invention, the left and right contact lenses are adapted to be contained in the respective lens containing portions that open upward in a horizontal state. Accordingly, during the contact lens sterilization process, the contact lens can be directly and easily put in or taken out from the lens containing portion. Moreover, owing to the contact preventing member, the present invention is capable of avoiding problems such as deposition of a heavy metal to the contact lens, damage to the contact lens, or the like, while stably performing the neutralization process with respect to the hydrogen peroxide solution by means of the metal catalyst.

### BRIEF DESCRIPTION OF THE DRAWINGS

FIG. 1 is a perspective view of a case main unit that constitutes a case for sterilizing contact lenses according to a first embodiment of the present invention.
FIG. 2 is a perspective view of a lid that constitutes the case for sterilizing contact lenses in cooperation with the case main unit of FIG. 1.
FIG. 3 is a perspective view of a catalyst that constitutes the case for sterilizing contact lenses in cooperation with the case main unit of FIG. 1 and the lid of FIG. 2.
FIG. 4 is a top plane view of the case main unit of FIG. 1.
FIG. 5 is a cross sectional view taken along line 5-5 of FIG. 4.
FIG. 6 is a cross sectional view taken along line 6-6 of FIG. 4.
FIG. 7 is a top plane view of the lid of FIG. 2.
FIG. 8 is a cross sectional view taken along line 8-8 of FIG. 7.
FIG. 9 is a cross sectional view taken along line 9-9 of FIG. 7.
FIG. 10 is an enlarged longitudinal cross sectional view of the catalyst in FIG. 3.
FIG. 11 is an enlarged transverse cross sectional view of the catalyst in FIG. 3, taken along line 11-11 of FIG. 10.
FIG. 12 is a view depicting a usage of the case for sterilizing contact lenses according to the first embodiment of the present invention which is constituted by the case main unit of FIG. 1, the lid of FIG. 2, and the catalyst of FIG.3.
FIG. 13 is a view depicting another usage of the case for sterilizing contact lenses of FIG. 12.
FIG. 14 is a perspective view of a case main unit that constitutes a case for sterilizing contact lenses according to a second embodiment of the present invention.
FIG. 15 is a top plane view of the case main unit of FIG. 14.
FIG. 16 is a cross sectional view taken along line 16-16 of FIG. 15.
FIG. 17 is a cross sectional view taken along line 17-17 of FIG. 15.
FIG. 18 is a cross sectional view taken along line 18-18 of FIG. 15.
FIG. 19 is a perspective view depicting a specific example of a catalyst that constitutes the case for sterilizing contact lenses in cooperation with the case main unit of FIG. 14.
FIG. 20 is a perspective view depicting another specific example of a catalyst employable instead of the catalyst of FIG. 19.
FIG. 21 is a top plane view depicting yet another specific example of a catalyst employable instead of the catalysts of FIG. 19 and FIG. 20.
FIG. 22 is a view depicting the catalyst of FIG. 21 installed.
FIG. 23 is a perspective view of another specific example of a catalyst for use in the case main unit of FIG. 1, which is employable instead of the catalyst of FIG. 3.
FIG. 24 is a view depicting yet another specific example of a catalyst employable instead of the catalyst of FIG. 23.
FIG. 25 is a perspective view of a case for sterilizing contact lenses according to a third embodiment of the present invention.
FIG. 26 is a top plane view of the case for sterilizing contact lenses of FIG. 25.
FIG. 27 is a perspective view of a case for sterilizing contact lenses according to a fourth embodiment of the present invention.
FIG. 28 is a longitudinal cross sectional view of the case for sterilizing contact lenses of FIG. 27.
FIG. 29 is a longitudinal cross sectional view of a case for sterilizing contact lenses according to a fifth embodiment of the present invention.

### EMBODIMENTS FOR CARRYING OUT THE INVENTION

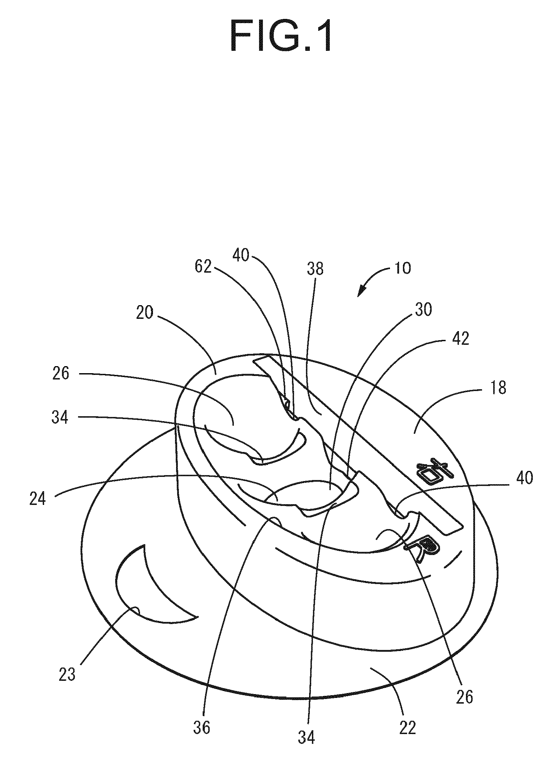

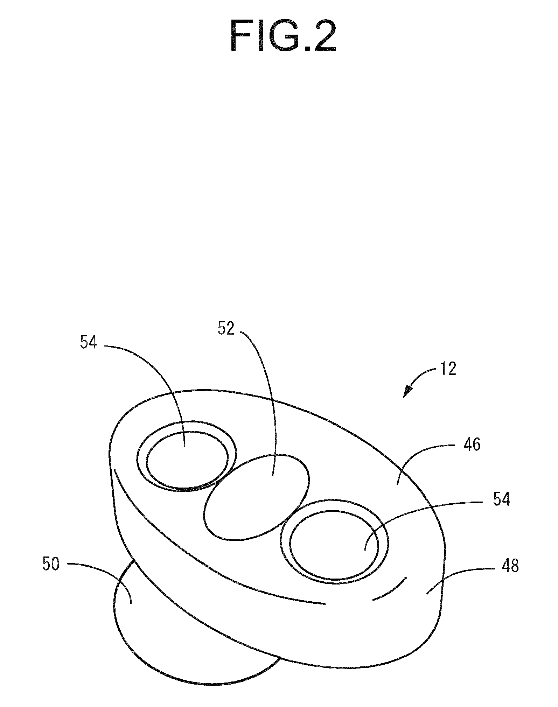

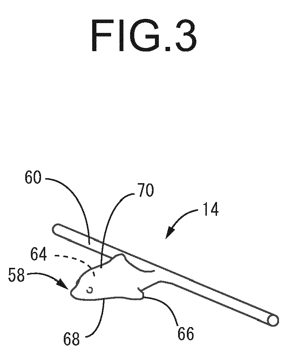

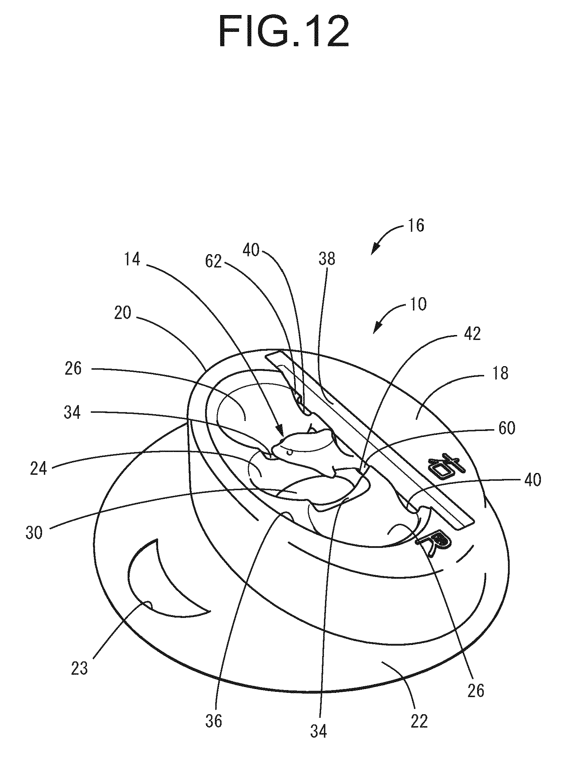

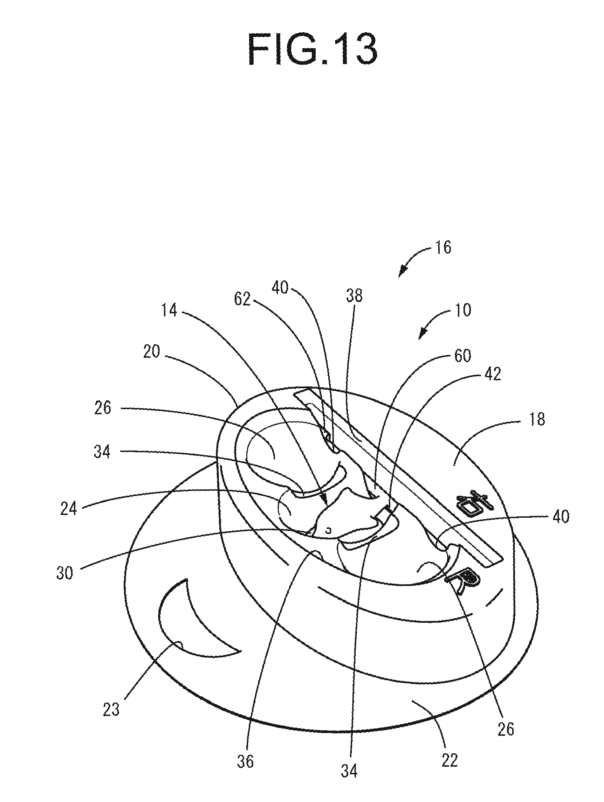

Following, embodiments of the present invention are described while referring to the drawings. First, FIG. 1 depicts a case main unit 10 which constitutes a case for sterilizing contact lenses, FIG. 2 depicts a lid 12, and FIG. 3 depicts a metal catalyst 14 for use in contact lens sterilization. The metal catalyst 14 of FIG. 3 is attached to the case main unit 10 of FIG. 1, and the lid 12 of FIG. 2 is mounted thereto in a reclosable manner, so that a case for sterilizing contact lenses 16 according to a first embodiment of the present invention (see FIGS. 12 and 13) is provided.

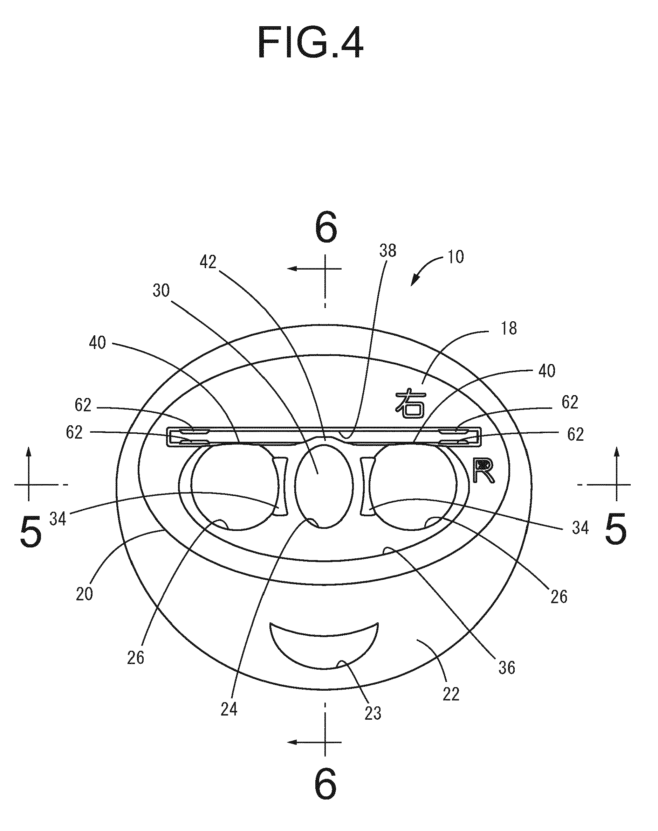

Described more specifically, the case main unit 10 shown in FIG. 1, as also depicted in FIGS. 4 through 6, is furnished with an oval-shaped upper base part 18 and a peripheral wall 20 extending downward from the outer peripheral edge of the upper base part 18. The case main unit 10 is a hollow body of inverted bowl shape that opens downward. In preferred practice, the case main unit 10 is integrally molded from a synthetic resin material such as polyethylene terephthalate or polypropylene. The case main unit 10 is transparent in its entirety, so that the contact lens, the metal catalyst 14 or the like contained within the case main unit 10 are visible from the outside through the case main unit 10.

Further, the case main unit 10 is furnished with a flange-shaped support plate 22 that flares toward the outer peripheral side from the lower end peripheral edge of the peripheral wall 20. Owing to this support plate 22, the case main unit 10 can be stably placed on a flat, horizontal support surface such as a table. Note that the support plate 22 has a hanging hole 23, and by inserting a hook rod or a hook through this hanging hole 23, it is possible for a user to dry the lens case every day. Also, a sales outlet or the like can suspend and stock the case main unit 10 and hence the case for sterilizing contact lenses 16.

Moreover, the upper base part 18 of the case main unit 10 has a catalyst containing portion 24 at approximately center section thereof. Furthermore, there are formed a pair of lens containing portions 26, 26 on opposite sides of the catalyst containing portion 24 in the major axis direction of the upper base part 18. The pair of lens containing portions 26, 26 respectively contains a contact lens for a left eye and a contact lens for a right eye. The pair of lens containing portions 26, 26 and the catalyst containing portion 24 are respectively formed as recesses that open onto the outside surface of the upper base part 18. The concave bottom surface of the lens containing portions 26, 26 is adapted to support the contact lens horizontally in the state where the convex side lens surface faces toward the bottom surface and the concave side lens surface faces toward the opening of the lens containing portions 26, 26. During the desired sterilization process of the contact lens, a hydrogen peroxide solution 44 is injected and stored in each of the catalyst containing portion 24 and the lens containing portions 26, 26. In addition, the respective left and right contact lenses are placed in the lens containing portions 26, 26, while the metal catalyst 14 described later is placed in the catalyst containing portion 24.

In particular, each of the lens containing portions 26, 26 is a semispherical recess that opens with a diameter greater than the outside diameter dimension of the contact lens to be treated. The catalyst containing portion 24 has an oval-shaped opening with its major axis coinciding with the minor axis direction of the upper base part 18 and has a bottom surface 30 of a flat surface. Note that the bottom surface 30 of the catalyst containing portion 24 is positioned higher (nearer to the opening) than the deepest part of the lens containing portions 26, 26, so that the catalyst containing portion 24 is shallower than the lens containing portions 26, 26. Additionally, the entire case main unit 10 is formed of a transparent material, including the wall which forms these lens containing portions 26, 26 and the catalyst containing portion 24. This arrangement provides a wall for visual observation permitting to observe from the outside the displacement state of the contact lens or the metal catalyst 14 contained therein. In the present embodiment, the bottom surface 30 of the catalyst containing portion 24 is positioned higher (nearer to the opening) than the deepest part of the lens containing portions 26, 26, so that the catalyst containing portion 24 is shallower than the lens containing portions 26, 26. However, the structure is not limited thereby. Specifically, it is possible to modify the depth or capacity of the catalyst containing portion 24 so as to adjust decomposition efficiency of the hydrogen peroxide solution, or adjust the amount of displacement of the catalyst 14 described later, or the like. In association therewith, the catalyst containing portion 24 may have a greater depth or a larger capacity than that of the lens containing portion 26.

Between the catalyst containing portion 24 and the neighboring lens containing portions 26, 26, there are formed dividing walls 32, 32 serving as a contact preventing member. This arrangement will prevent the contact lens contained in the lens containing portions 26, 26 from coming into contact with the metal catalyst 14 contained in the catalyst containing portion 24. The dividing walls 32, 32 have a pair of adjacent communication passages 34, 34 each having U-shaped cross section, serving as a mutual communication passage. Specifically, through these adjacent communication passages 34, 34, the catalyst containing portion 24 communicates with the lens containing portions 26, 26 on its opposite sides. When the hydrogen peroxide solution 44 is injected into the lens containing portions 26, 26 and the catalyst containing portion 24, the adjacent communication passages 34, 34 allow the hydrogen peroxide solution 44 to flow among the lens containing portions 26, 26 and the catalyst containing portion 24. With this arrangement, the pair of the lens containing portions 26, 26 are interconnected via the adjacent communication passages 34, 34 and the catalyst containing portion 24. Note that the depth of the adjacent communication passage 34 is shallower than that of the catalyst containing portion 24 or the lens containing portion 26. With respect to each of the pair of the adjacent communication passages 34, 34 that connect the catalyst containing portion 24 and the lens containing portions 26, 26, one aperture opens onto the rim of the opening of the catalyst containing portion 24 so as to opposed to each other in the lateral direction. Meanwhile, the other aperture of each of the adjacent communication passages 34, 34 opens onto the rim of the opening of the lens containing portion 26 with a length equal to 1/5 or more of the circumference of the outside edge of the lens containing portion 26. However, if a support rod 60 of the catalyst depicted in FIG. 3 also serves as a catalyst, the sum of the lengths of the adjacent communication passage 34 and an end connection passage 40 will be equal to 1/5 or more of the circumference of the outside edge of the lens containing portion 26.

Moreover, the case main unit 10 includes on its upper base part 18 a common recess 36 of generally oval shape that spreads so as to surround a periphery of the catalyst containing portion 24 and the lens containing portions 26, 26. The common recess 36 has a generally oval shape that is slightly smaller than the upper base part 18 with a sloping bottom surface that becomes progressively deeper from the outer peripheral edge toward the center. The catalyst containing portion 24, the lens containing portions 26, 26, and the adjacent communication passages 34, 34 open onto the bottom surface of the common recess 36.

The case main unit 10 further includes on its upper base part 18 a parallel communication groove 38 serving as a parallel communication passage that abuts against one end edge of the common recess 36 (the upper end edge in FIG. 4) in the minor axis direction. The parallel communication groove 38 extends in a straight line in the major axis direction of the upper base part 18 (the lateral direction in FIG. 4), which coincides with the direction of array of the catalyst containing portion 24 and the pair of the lens containing portions 26, 26. The lengthwise opposite ends of the parallel communication groove 38 provide the end connection passages 40, 40 at the dividing wall between the parallel communication groove 38 and the respective lens containing portions 26, 26. Meanwhile, the lengthwise middle portion of the parallel communication groove 38 provide a middle connection passage 42 at the dividing wall between the parallel communication groove 38 and the catalyst containing portion 24.

Note that in the present embodiment, the depth of the parallel communication groove 38 is shallower than that of the catalyst containing portion 24 and the lens containing portion 26 while deeper than that of the adjacent communication passage 34. The depth of the middle connection passage 42 is deeper than that of the end connection passage 40, 40 and the adjacent communication passage 34 while approximately equal to that of the parallel communication groove 38. The depth of the end connection passage 40 is shallower than that of the middle connection passage 42 while approximately equal to that of the adjacent communication passage 34. However, the depth or capacity of each of the parallel communication groove 38, the middle connection passage 42, and the end connection passage 40 is not limited by the mode of the present embodiment. By adjusting these values mutually to one another or relatively to that of the catalyst containing portion 24 or the lens containing portion 26 etc., it is possible to adjust fluid flow efficiency among these areas or to adjust decomposition efficiency of the hydrogen peroxide solution within the catalyst containing portion 24. In addition, the width and depth of the adjacent communication passage 34 and the parallel communication groove 38 are established so as to be capable of preventing movement of the contact lens contained in the lens containing portion 26 toward the catalyst containing portion 24 or the parallel communication groove 38. Specifically, in the present embodiment, a passage constricting member is provided including the dividing wall 32 which divides the lens containing portion 26 and the catalyst containing portion 24, and the adjacent communication passage 34. This arrangement permits flow of the hydrogen peroxide solution 44 while preventing movement of the contact lens toward the catalyst containing portion 24 and movement of the metal catalyst 14 toward the lens containing portion 26. Note that the structure of the passage constricting member is not limited in particular to those by reducing the passage width or the passage depth, provided that it can be able to prevent movement of the contact lens and the metal catalyst 14. For example, it would also be acceptable to separately provide a net-shaped or lattice-shaped fence in order to prevent movement of the contact lens and the metal catalyst 14.

Furthermore, the parallel communication groove 38 communicates with both of the catalyst containing portion 24 and the lens containing portions 26, 26. Accordingly, the catalyst containing portion 24 and the lens containing portions 26, 26 are interconnected via the parallel communication groove 38. With the catalyst containing portion 24 and the lens containing portions 26, 26 interconnected via the adjacent communication passage 34, the parallel communication groove 38, the end connection passages 40, 40, and the middle connection passage 42, the catalyst containing portion 24 and the lens containing portions 26, 26 are not only connected in series but also interconnected so as to form an annular circulation passage. Specifically, the annular circulation passage is constructed as follows: "catalyst containing portion 24 → adjacent communication passage 34(left) → lens containing portion 26(left) → end connection passage 40(left) → parallel communication groove 38 → end connection passage 40(right) → lens containing portion 26(right) → adjacent communication passage 34(right) → catalyst containing portion 24"; or "catalyst containing portion 24 → adjacent communication passage 34(left) → lens containing portion 26(left) → end connection passage 40(left) → parallel communication groove 38 → middle connection passage 42 → catalyst containing portion 24"; or the like.

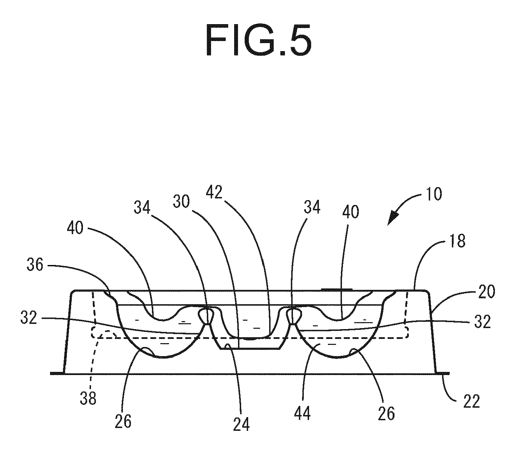

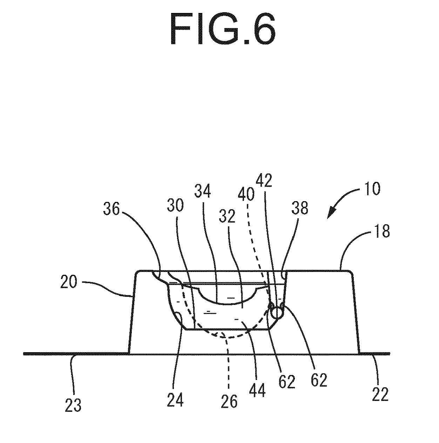

At the time of the sterilization process of the contact lens, the hydrogen peroxide solution 44 is injected and stored in the catalyst containing portion 24 and the lens containing portions 26, 26 (see FIGS. 5 and 6). As the hydrogen peroxide solution 44 to be injected therein, a 3% solution or the like, which is commercially available for contact lens sterilization, would be preferably employed. The hydrogen peroxide solution 44 is injected and stored so as to not only enter the catalyst containing portion 24 and the lens containing portions 26, 26 but also go around all of the adjacent communication passages 34, the parallel communication groove 38, the end connection passages 40, 40, and the middle connection passage 42, with the volume such that the solution surface becomes higher than the bottom surface of any of those. The catalyst containing portion 24 and the lens containing portions 26, 26 communicate with one another via the adjacent communication passages 34, the parallel communication groove 38, the end connection passages 40, 40 and the middle connection passage 42 so as to provide the annular circulation passage. Accordingly, the hydrogen peroxide solution 44 stored in the catalyst containing portion 24 and the lens containing portions 26, 26 is allowed to flow among these containing portions.

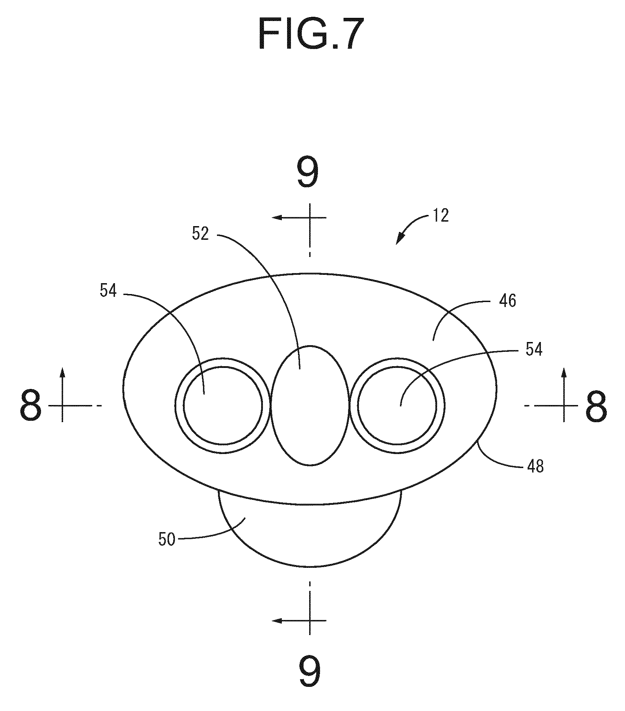

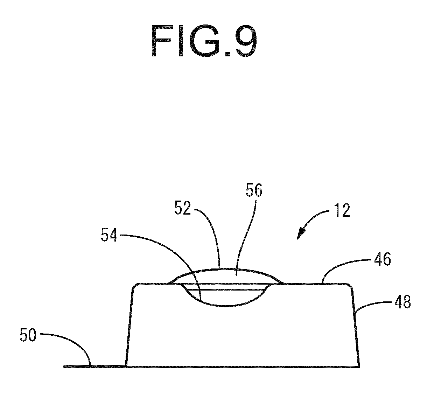

Meanwhile, as shown in FIGS. 7 through 9, the aforementioned lid 12 as depicted in FIG. 2 has a cover shape that corresponds to the case main unit 10. Specifically, the lid 12 is furnished with an oval-shaped upper base part 46 and a peripheral wall 48 extending downward from the outer peripheral edge of the upper base part 46. The lid 12 is a hollow body of inverted bowl shape that opens downward. Similarly to the case main unit 10, the lid 12 is preferably an integrally molded component of synthetic resin material. However, it would also be possible for the lid 12 to be thin-walled compared to the case main unit 10, and to be molded by injection molding of a resin material or hot-pressing of a resin film or the like, for example. In order to visually observe the catalyst containing portion 24 and the lens containing portions 26, 26 of the case main unit 10 from the outside even when the case main unit 10 is covered by the lid 12, it is particularly preferred for the lid 12 to be transparent at least in a part that covers the catalyst containing portion 24, and more preferably, as far as a part that covers the lens containing portions 26, 26.

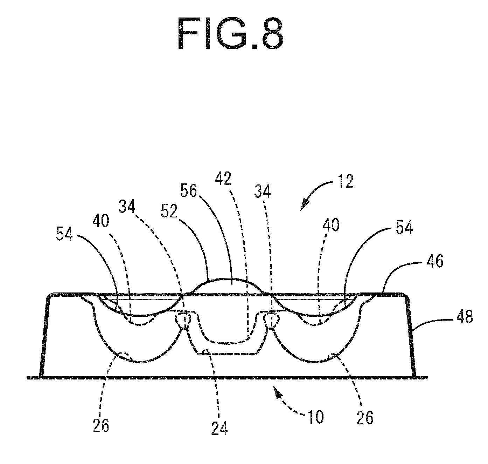

The lid 12 has a size slightly larger than that of the case main unit 10 by approximately the wall thickness of the lid 12. As depicted in FIG. 8 together with the case main unit 10 shown by the imaginary line, the lid 12 is adapted to be superposed and detachably mounted onto the case main unit 10 so that the concave side face of the lid 12 overlaps the convex side face of the case main unit 10. By so doing, both of the catalyst containing portion 24 and the lens containing portions 26, 26 will be covered with the lid 12.

The lid 12 is kept mounted onto the case main unit 10 by means of its peripheral wall 48 being fitted externally onto the peripheral wall 20 of the case main unit 10. However, it would also be acceptable to provide concave/convex detents or the like to the two peripheral walls 48, 20 in order to avoid unexpected detachment of the lid 12 from the case main unit 10. In addition, the lid 12 is furnished with a pull tab 50 of crescent shape that extends toward the outer peripheral side from a part of the lower end peripheral edge of the peripheral wall 48. By gripping by fingers the pull tab 50 of the lid 12 superposed against the case main unit 10, the lid 12 can be easily detached from the case main unit 10. Note that with the lid 12 superposed against the case main unit 10, there is formed a tiny gap between the lid 12 and the case main unit 10, which serves as a gas discharge passage. Through the gap, the oxygen gas generated in association with decomposition of the hydrogen peroxide solution 44 can be discharged.

Besides, the lid 12 includes on its upper base part 46 a catalyst lid 52 at its generally center, and a pair of lens lids 54, 54 serving as inward convex portions at opposite sides of the catalyst lid 52 in the major axis direction of the upper base part 46. The catalyst lid 52 has a convex dome shape that bulges to the outer surface side of the upper base part 46. On the other hand, the lens lids 54, 54 each have a concave dome shape that bulges to the inner surface side. Note that all of these catalyst lid 52 and the lens lids 54, 54 are transparent, so that it is possible to observe from the outside the condition of displacement of the metal catalyst 14 contained in the catalyst containing portion 24, or the condition of the contact lenses contained in the lens containing portions 26, 26.

By means of the lid 12 being superposed against and attached to the case main unit 10, the catalyst lid 52 and the lens lids 54, 54 of the lid 12 are placed on the respective openings of the catalyst containing portion 24 and the lens containing portions 26, 26 and cover them. In this state, there is formed a cover space 56 above the opening of the catalyst containing portion 24 which is covered by the catalyst lid 52 projecting outward. Meanwhile, the lens lids 54 projecting inward is inserted into the respective lens containing portions 26. With this arrangement, the center portion of the lens lid 54 is pushed from the surface to inside of the hydrogen peroxide solution 44, which is injected in the lens containing portion 26.

Moreover, the lens lids 54, 54 of the lid 12 provide a temporary lens platform by its convex face or concave face. In preferred practice, the curvature radius of the convex face or concave face of the lens lids 54, 54 is established to the value approximate to a general curvature of the convex face or concave face of a contact lens. By so doing, with the lid 12 detached from the case main unit 10 and placed on the flat, horizontal support face, the user of the contact lens can temporarily rest the removed contact lens onto the convex face or concave face of the lens lid 54, before or after the sterilization process. In order to use the convex face of the lens lid 54 as the temporary lens platform, the lid 12 will be inverted and placed on the table or the like so as to open upward. On the contrary, when the lid 12 is not inverted and placed on the table or the like so as to open downward, the concave face of the lens lid 54 can be used as the temporary lens platform.

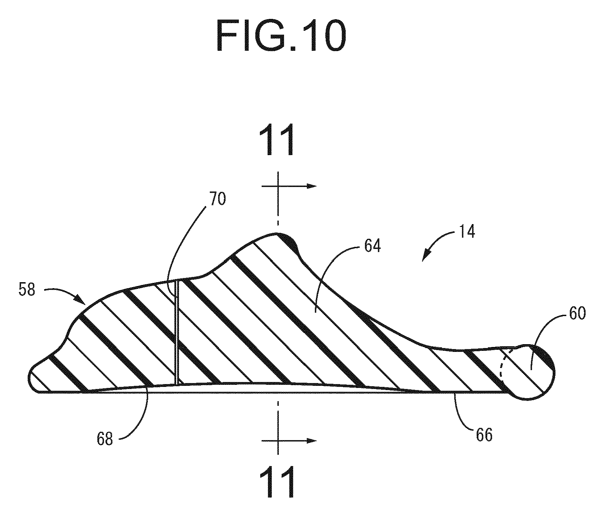

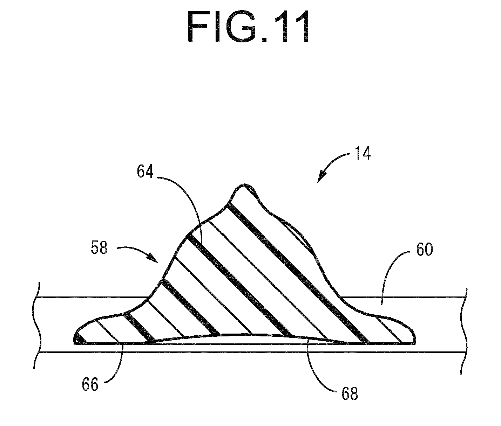

Furthermore, the metal catalyst 14 as depicted in FIG. 3 has a block shape which is appropriately formed, as shown in enlarged views in FIGS. 10 and 11. In the present embodiment in particular, the metal catalyst 14 has contours that resemble a dolphin in expectation of attracting the interest of the observers.

The metal catalyst 14 has a size so as to be inserted into the catalyst containing portion 24 of the case main unit 10 and displaceable within the catalyst containing portion 24. In particular, the height dimension of the metal catalyst 14 is smaller than the depth dimension of the catalyst containing portion 24. More preferably, the metal catalyst 14 has a size with its height dimension being smaller than the solution depth of the hydrogen peroxide solution 44 injected in the catalyst containing portion 24 so as to be completely sunk within the hydrogen peroxide solution 44.

Moreover, the metal catalyst 14 has at its outer peripheral edge a support rod 60 extending in a straight line. In the present embodiment in particular, since the metal catalyst 14 has elongated contours that resemble a dolphin, the support rod 60 is integrally formed in a mode that projects from the lengthwise one end portion, which corresponds to the tail of the dolphin, toward the widthwise opposite sides of the dolphin. That is, the metal catalyst 14 is provided so as to project from the lengthwise middle section of the support rod 60 in the axis-perpendicular direction.

As depicted in FIGS. 12 and 13, the support rod 60 is contained in the parallel communication groove 38 of the case main unit 10 while the metal catalyst 14 extends through the middle connection passage 42 of the case main unit 10 toward inside of the catalyst containing portion 24 and is contained therein. The support rod 60 is contained in the base part of the parallel communication groove 38 of the case main unit 10, and the parallel communication groove 38 has retaining projections 62, 62 formed projecting from its opposed walls. Owing to the mating action of the retaining projections 62, 62 against the support rod 60, the support rod 60 is prevented from becoming dislodged out of the parallel communication groove 38. Additionally, while contained in the parallel communication groove 38, the support rod 60 is readily permitted to rotate about its center axis. In association with the rotation of the support rod 60, the metal catalyst 14 is readily permitted to displace in the emerging/submerging direction within the catalyst containing portion 24.

As will be apparent from the above description, in the present embodiment, the support rod 60 formed to the metal catalyst 14 provides a mating portion against the case main unit 10, while the parallel communication groove 38 and the retaining projections 62, 62 projecting from its opposed walls provide a retaining portion for mating and retaining the support rod 60. On the basis of the mating action between the support rod 60 and the retaining projections 62, 62, a reserving member is defined for permitting displacement of the metal catalyst 14 within the catalyst containing portion 24 in emerging/submerging direction while preventing unnecessary detachment of the metal catalyst 14 from the case main unit 10. With this arrangement, after the sterilization process, even during discharge of the hydrogen peroxide solution 44 from the catalyst containing portion 24 or washing of the case main unit 10 or the like, the metal catalyst 14 will be reserved within the catalyst containing portion 24.

Additionally, the metal catalyst 14, at least a part of its surface which comes into contact with the solution (hydrogen peroxide solution) 44 when being immersed within the hydrogen peroxide solution 44, is formed of a metallic material that catalyzes decomposition of the hydrogen peroxide solution 44. With this arrangement, the metal catalyst 14 is a metal catalyst that catalyzes decomposition reaction of the hydrogen peroxide solution 44. As the metallic material, while it is possible to employ any of those conventionally known, a metal that is able to exhibit a stable catalysis during a contact lens sterilization process of multiple times would preferably be employed. As a specific example, at least one of metals and metal oxides thereof selected from the group consisting of platinum, silver, palladium, copper, manganese, cobalt, and aluminum is employed as the metallic material of the metal catalyst 14.

Note that it is not necessary for the metal catalyst 14 to be entirely formed of the above-mentioned metal catalyst. For example, the interior of the metal catalyst 14 which does not come into contact with the hydrogen peroxide solution 44, or a part of its surface which comes into contact with the hydrogen peroxide solution 44 etc. are allowed to be formed of a material that does not exhibit catalysis. This arrangement makes it possible for instance to adjust the mass (M) of the metal catalyst 14 or to attain a greater degree of freedom in molding the shape etc. of the metal catalyst 14. As a specific example, as the material that is combined with the above-described catalyst material so as to form the metal catalyst 14 and does not exhibit catalysis, it is possible to use at least one type of material selected from plastic, metal, glass, and ceramic. Furthermore, as the aforementioned plastic, preferably employed is at least one type of material selected from the group consisting of acrylonitrile-butadiene-styrene resin, polyurethane, modified polyphenylene ether, polystyrene, polycarbonate, polyethylene terephthalate, polybutylene terephthalate, polyvinyl chloride, polyetherimide, polysulfone, polymethylmethacrylate, and copolymer resins thereof.

In particular, as the metal catalyst 14 of composite structure, for example, employed is the metal catalyst 14 in which a base material 64 molded from a material that does not exhibit catalysis as described above into an appropriate shape (for example, a dolphin shape as shown) is used and a catalyst layer 66 serving as a metal coat and formed of a material that exhibits catalysis as described above is adhered to the surface of the base material 64. The catalyst layer 66 can be formed with a suitable thickness by a thin film forming technique of conventionally known such as plating or sputtering so as to cover the surface of the base material 64.

In addition, the metal catalyst 14 is arranged so as to have an applicable buoyancy (F) that acts within the hydrogen peroxide solution and undergo displacement in the emerging/submerging direction in association with progress of neutralization (decomposition reaction) of the hydrogen peroxide solution 44 while being immersed therein within the catalyst containing portion 24. In preferred practice, a ratio M/F between the mass (M) of the metal catalyst 14 and the buoyancy (F) exerted on the metal catalyst 14 within the hydrogen peroxide solution is set to more than 1 and does not exceed 2. Note that the buoyancy (F) is a force that is exerted on the catalyst main body 58 by itself, and does not include an external force that is exerted on the metal catalyst 14 by the oxygen gas (bubbles) generated in association with decomposition reaction of the hydrogen peroxide solution 44, for example.

Meanwhile, the buoyancy (F) can be adjusted by various measures. For example, it is possible to adjust the buoyancy by changing the material of the base material 64 to a selected material of an appropriate specific gravity. It is particularly preferred to employ a material having a specific gravity that is greater than 1 and does not exceed 3 as the base material 64.

Alternatively, it would also be possible to adjust the buoyancy by imparting a hollow structure to the metal catalyst 14 so as to have voids inside. The voids can be formed to applicable positions in desirable number by blow molding or lost-core molding, for example. It could instead be acceptable to impart a foam structure including closed cells by foam molding.

While a material or mass of the support rod 60 integrally formed with the metal catalyst 14 is not limited in particular, it is desirable that the support rod 60 be molded from the same material as the metal catalyst 14 in consideration of corrosion resistance, formability, strength or the like. Since the support rod 60 will also be immersed in the hydrogen peroxide solution 44 within the parallel communication groove 38, by using a material that exhibits catalysis in order to form at least the surface of the support rod 60, the neutralization speed of the hydrogen peroxide solution 44 can be adjusted in cooperation with the metal catalyst 14. At that time, by forming a plurality of irregularities on the surface of the support rod 60, there will be a greater degree of freedom in adjusting the surface area and hence the catalysis of the support rod 60. Moreover, as described above, the support rod 60 permits the metal catalyst 14 to undergo displacement in the emerging/submerging direction on the basis of its rotational displacement. Accordingly, it would also be possible for example to form a plurality of projections of ring shape projecting from the outside peripheral face of the support rod 60 and extending in the circumferential direction so as to be arranged at a suitable intervals in the axial direction. With this arrangement, the contact surface of the support rod 60 with respect to the inside face of the parallel communication groove 38 and hence the rotational resistance will be reduced.

When the metal catalyst 14 is immersed within the hydrogen peroxide solution 44, the decomposition of the hydrogen peroxide solution 44 will start owing to the catalysis of the catalyst layer 66 formed on the surface of the metal catalyst 14. In association therewith, the oxygen gas will be generated and come into contact with the metal catalyst 14 while being discharged from the surface of the hydrogen peroxide solution 44 to outside as bubbles. This will exert a buoyancy on the metal catalyst 14 as the external force. By utilizing the buoyancy of the oxygen gas, the metal catalyst 14 is adapted to undergo displacement within the hydrogen peroxide solution 44 depending on the extent of the progress of decomposition reaction of the hydrogen peroxide.

Besides, in the present embodiment, the metal catalyst 14 has on its surface a plurality of concave portions and convex portions, which configurate the head, body, tail, back, belly, mouth, pectoral fin, dorsal fin, caudal fin or the like so as to have contours of dolphin shape overall. In particular, the metal catalyst 14 has a hollow 68 of shallow concave shape serving as a concave portion that extends over substantially its entire bottom surface including its belly and opens downward. Owing to these concave portions and convex portions, the metal catalyst 14 has a sufficient surface area and is able to carry out the catalytic reaction by the catalyst layer 66 in an applicable manner. It is desirable that the metal catalyst 14 have a surface area of between 3 and 30 cm² per 10 ml of the hydrogen peroxide solution 44. In addition, the metal catalyst 14 further includes a discharge passage 70 that vertically and straightly perforates the metal catalyst 14 near its center. The lower end of the discharge passage 70 opens near the center of the hollow 68. Note that the number or shape of the hollow 68 and the discharge passage 70 are not limited to those shown in the drawings but may be desirably established.

By so doing, when the metal catalyst 14 is immersed within the hydrogen peroxide solution 44, the oxygen gas generated during the decomposition reaction of the hydrogen peroxide solution 44 will be stored in the hollow 68, whereby the buoyancy of the oxygen gas will more effectively be exerted on the metal catalyst 14 as the external force. The buoyancy exerted on the metal catalyst 14 by means of the oxygen gas stored in the hollow 68 can be adjusted by redesigning the hollow 68 in its capacity or position (in particular, the distance apart from the support rod 60 that coincides with the center axis of swinging displacement in the emerging/submerging direction). The excess oxygen gas will overpass the peripheral wall of the hollow 68 and be discharged upward from the outer periphery of the metal catalyst 14.

When the decomposition reaction of the hydrogen peroxide solution 44 is substantially terminated and the neutralization is completed, generation of oxygen gas will be stopped. However, at that time, the buoyancy of the oxygen gas continues to be exerted on the metal catalyst 14 as long as the oxygen gas is stored in the hollow 68. Meanwhile, the oxygen gas stored in the hollow 68 continues to be discharged through the discharge passage 70 to outside in a prescribed limiting manner.

In preferred practice, the outflow of the oxygen gas from the hollow 68 through the discharge passage 70 per unit time is set smaller than the inflow of the oxygen gas into the hollow 68 per unit time during decomposition reaction of the hydrogen peroxide solution 44. With this arrangement, at the time which is substantially simultaneous with or behind the completion of the neutralization process by means of decomposition reaction of the hydrogen peroxide solution 44, the amount of oxygen gas stored in the hollow 68 becomes insufficient for the metal catalyst 14 to emerge. By so doing, it is possible to keep the metal catalyst 14 emerging during the decomposition reaction of the hydrogen peroxide solution 44 while having the metal catalyst 14 submerge due to dissipation of the buoyancy when the neutralization of the hydrogen peroxide solution 44 is substantially completed. Note that the outflow of the oxygen gas through the discharge passage 70 per unit time can be adjusted by modifying discharge resistance of the oxygen gas through the discharge passage 70. As a specific example, modifying the cross-sectional area or the cross-sectional shape of the discharge passage 70, or modifying the properties of the surface of the discharge passage 70 such as the extent of hydrophobic property or hydrophilic property may be employed.

When carrying out a contact lens sterilization process by using the case for sterilizing contact lenses 16 constructed as above, the following method will be employed. First, the user of the contact lens places the case main unit 10 on the table or the like with the lid 12 detached. Then, the user injects and stores the hydrogen peroxide solution 44 prepared in advance for a lens sterilization into a solution containing portion which includes the catalyst containing portion 24 and the lens containing portions 26, 26 etc.

In addition, before or after the injection of the hydrogen peroxide solution 44, the user set the metal catalyst 14 in the case main unit 10. By so doing, the support rod 60 provided to the metal catalyst 14 is placed in the parallel communication groove 38 of the case main unit 10 and positioned at the deepest part thereof. The metal catalyst 14 is disposed within the catalyst containing portion 24 thereby. With this arrangement, the metal catalyst 14 is adapted to come into contact with the hydrogen peroxide solution 44 stored in the catalyst containing portion 24.

Besides, before or after the injection of the hydrogen peroxide solution 44 or setting of the metal catalyst 14, the user removes the respective contact lenses from his or her right eye and left eye, and places the removed contact lenses in the lens containing portion 26 of the case main unit 10.

In the case where the user injects the hydrogen peroxide solution 44 in the lens containing portion 26 and then removes and places the contact lens in the lens containing portion 26, it is desirable that before placing the removed contact lens in the lens containing portion 26, the user temporarily rest the removed contact lens on the convex face or the concave face of the lens lid 54 serving as the temporary lens platform, which is provided to the lid 12 detached from the case main unit 10 and placed on the table or the like. Specifically, if one of the left/right contact lenses is removed and placed in one lens containing portion 26 and then the other contact lens is removed and placed in the other lens containing portion 26, when the first-removed contact lens is placed in the lens containing portion 26, the hydrogen peroxide solution 44 injected in the lens containing portion 26 may touch the user's finger. Consequently, there is a risk that the hydrogen peroxide solution 44 adhered to the finger may touch the user's eye and cause troubles such as irritation when the user removes the other contact lens. Therefore, as described above, it is preferable to utilize the convex face or the concave face of the lens lid 54 for removing the contact lenses from both eyes and temporarily resting them, and then to place the two contact lenses in the two lens containing portions 26, 26 sequentially. By so doing, even if the hydrogen peroxide solution 44 touches the finger when the user places the contact lens in the lens containing portion 26, it is possible to prevent the hydrogen peroxide solution 44 from coming into the user's eye.

Thereafter, by laying the lid 12 on the case main unit 10 and attaching thereto, the sterilization process of the contact lens will be started.

Specifically, since the contact lens for the right eye and the contact lens for the left eye contained within the lens containing portions 26, 26 are immersed in the hydrogen peroxide solution 44, the contact lens is subjected to the sterilization process for a prescribed time period. Also, since the metal catalyst 14 is immersed within the hydrogen peroxide solution 44, the oxygen gas is generated in accordance with the progress of the decomposition reaction of the hydrogen peroxide solution 44. Besides, at the beginning of the sterilization process, as depicted in FIG. 12, within the catalyst containing portion 24 of the case main unit 10, the metal catalyst 14 contained within the hydrogen peroxide solution 44 displaces upward and emerges by means of its own buoyancy and the buoyancy exerted by the generated oxygen gas (the external force). In preferred practice, as shown in the drawings, the upper portion of the metal catalyst 14 juts out and is exposed from the solution surface. Note that even if the lid 12 is superposed on the case main unit 10, since the catalyst lid 52 that covers the catalyst containing portion 24 has a hollow, convex dome shape and bulges outward, there is a sufficient inner space above the catalyst containing portion 24 for permitting the metal catalyst 14 to emerge and jut out.

In this state, along with the sterilization process of the contact lens by means of the hydrogen peroxide solution 44, the decomposition reaction of the hydrogen peroxide solution 44 progresses by means of the catalysis. At that time, the lens lids 54 of the lid 12 is inserted into the lens containing portions 26, 26 of the case main unit 10, and kept pushed into the hydrogen peroxide solution 44. With this arrangement, the contact lens contained in the lens containing portion 26 is pushed into the solution by the lens lid 54, and is completely immersed within the hydrogen peroxide solution 44. This will stably carry out the desired sterilization process. Concomitantly, the hydrogen peroxide solution 44 flows out from the lens containing portions 26, 26 by the amount equal to the capacity of the lens lids 54, 54 that is pushed into the solution. This flowing out of the hydrogen peroxide solution 44 will raise the solution surface of the other solution containing portions, whereby the solution surface of the catalyst containing portion 24 rises. By so doing, even if the amount of the hydrogen peroxide solution 44 injected into the solution containing portion is small, the solution surface of the catalyst containing portion 24 will reach a sufficient level. Thus, the hydrogen peroxide solution 44 reliably comes into contact with the metal catalyst 14, realizing emerging action and decomposition reaction of the metal catalyst 14.

After the sterilization process for a prescribed time period when the decomposition reaction of the hydrogen peroxide solution 44 is terminated, generation of the oxygen gas stops and the buoyancy exerted on the metal catalyst 14 by the oxygen gas dissipates. As a result, as depicted in FIG. 13, the metal catalyst 14 submerges. In particular, the metal catalyst 14 is placed in abutment with the bottom surface of the catalyst containing portion 24 so as not to be exposed from the surface of the hydrogen peroxide solution 44 but be completely immersed therein. Specifically, in the present embodiment, during the decomposition of the hydrogen peroxide solution 44 the metal catalyst 14 displaces upward and emerges due to the buoyancy exerted by the oxygen gas. On the other hand, when decomposition reaction stops, the metal catalyst 14 becomes held in non-displacement state from the emerging state, and submerges. Accordingly, after a prescribed time period has passed from the start of the sterilization process, the user of the contact lens visually checks that the metal catalyst 14 has submerged at the bottom surface of the catalyst containing portion 24, and is able to easily confirm that the decomposition reaction of the hydrogen peroxide solution 44 has been completed. Therefore, the user can confirm that the hydrogen peroxide solution 44 has been decomposed and completely changed into water, and thereafter safely take out the sterilized contact lenses.

In this way, at the start of the contact lens sterilization, the user visually observes that the bubbles (oxygen gas) generate around the metal catalyst 14 and that the metal catalyst 14 emerges so as to partially jut out from the solution surface. This enables the user to check that the hydrogen peroxide solution 44 is properly injected and the sterilization process has normally started. In particular, since the emergence of the metal catalyst 14 that resembles a dolphin out of the solution surface attracts the interest of the user and delights him/her, it can be expected that the user actively performs the observation. It can be further expected that the user is motivated to regularly carry out the contact lens sterilization process by his or her voluntary will. As a result, occurrence of eye diseases caused by wearing the polluted contact lens will be avoided, thereby accelerating using of good contact lens.

In addition, since the catalyst containing portion 24 is formed separately from the lens containing portions 26, 26, the capacity of the catalyst containing portion 24 and hence the surface area of the metal catalyst 14 can be sufficiently obtained, thereby effectively achieving the desired catalysis. Moreover, the opening of the catalyst containing portion 24 is formed separately from the openings of the lens containing portions 26, 26. Accordingly, it is possible to readily check the emergence/submergence of the metal catalyst 14 within the hydrogen peroxide solution 44 without deteriorating ease of placing in and taking out the contact lens with respect to the lens containing portions 26, 26. Also, the metal catalyst 14 partially juts out from the solution surface during its emergence, making it even easier to be observed.

Besides, the catalyst containing portion 24 and the lens containing portions 26, 26 that separately open from one another are interconnected via the adjacent communication passages 34, 34, the parallel communication groove 38, or the like. Moreover, the communication of these containing portions 24 and 26, 26 is not merely a serial one having the opposite passage ends, but the one forming an annular circulation passage having no passage end. With this arrangement, the decomposition reaction of the hydrogen peroxide solution 44 by means of the metal catalyst 14 contained in the catalyst containing portion 24 can easily spread out into the lens containing portions 26, 26. Thus, the neutralization will progress more uniformly over the entire hydrogen peroxide solution 44 contained in the solution containing portion including the catalyst containing portion 24 and the lens containing portions 26, 26. The desired sterilization effect as well as stability of the required time for completion of the neutralization or the like can be obtained thereby.

Moreover, the change of the amount of the oxygen gas generated in association with progress of the decomposition reaction of the hydrogen peroxide solution 44 or the change of the position of the generated oxygen gas will change the buoyancy exerted on the metal catalyst 14. In association with this change in buoyancy, the metal catalyst 14 undergoes displacement within the hydrogen peroxide solution 44, so that the hydrogen peroxide solution 44 will be stirred and made to flow. Accordingly, the uniform hydrogen peroxide solution 44 within the solution containing portion including the catalyst containing portion 24 and the lens containing portions 26, 26 as described above can be more effectively obtained.

Furthermore, the pair of lens containing portions 26, 26 are interconnected via each of the communicating grooves 34, 38 and the connection passages 40, 42. Additionally, both of the lens containing portions 26, 26 open onto the bottom surface of the common recess 36. Thus, when the user's finger enters the one lens containing portion 26 and takes out the contact lens, the solution pushed out of the one lens containing portion 26 can rapidly move to another area such as the other lens containing portion 26. Therefore, the solution pushed out of the lens containing portion 26 by the finger will effectively be prevented from spilling out of the common recess 36.

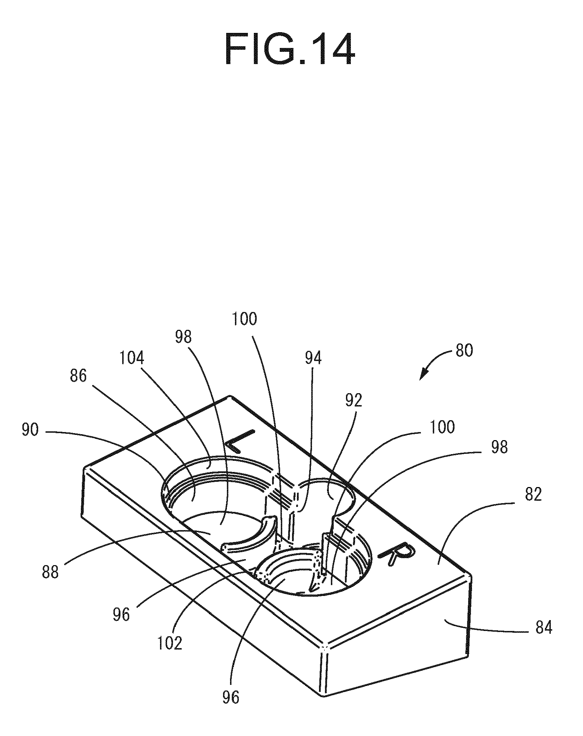

Next, FIG. 14 depicts a case main unit 80 which constitutes a case for sterilizing contact lenses according to a second embodiment of the present invention.

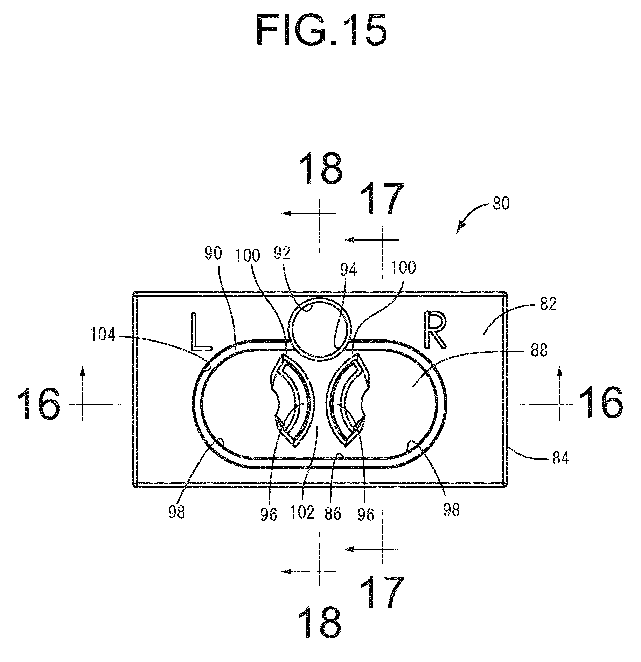

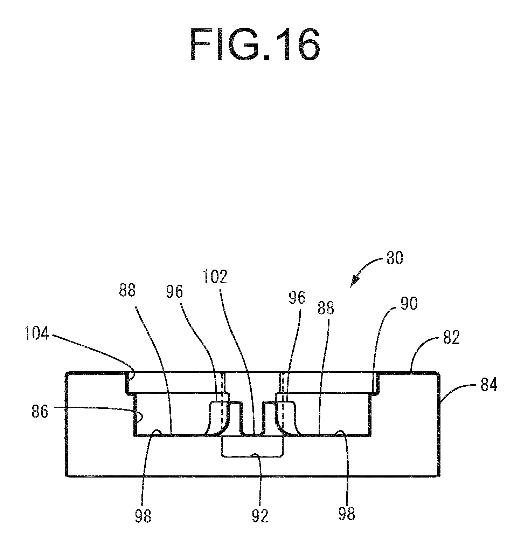

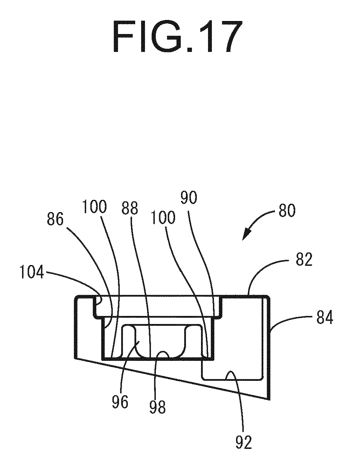

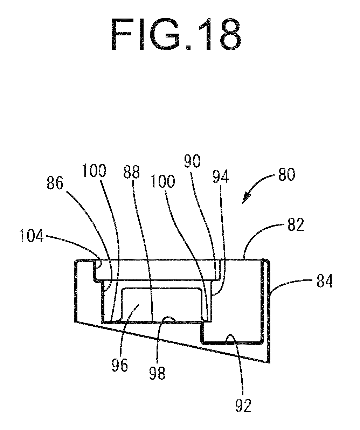

As depicted in FIGS. 15 through 18, the case main unit 80 is furnished with an upper base part 82 of rectangular shape and a peripheral wall 84 that extends downward from the outer peripheral edge of the upper base part 82 so as to have a hollow body of inverted bowl shape opening downward. The peripheral wall 84 is made higher at its long-side portion on the far side rather than at its long-side portion on the near side. With this arrangement, the upper base part 82 has a tilted surface that progressively tilts downward from the far side toward the near side. The user of the contact lens operates at the near side (the lower side in FIG. 15) of smaller height.

The case main unit 80 further includes a solution containing portion 86 of concave shape opening upward at the center section of the upper base part 82. The solution containing portion 86 has an oval shape (an elliptical shape) in plan view, and a bottom surface 88 thereof is a flat surface extending horizontally. In the peripheral wall inner surface of the solution containing portion 86, there is formed a step 90 extending continuously about the entire circumference in the circumferential direction at the middle section in the depth direction near the opening. The upper side (the opening side) of the step 90 is expanded.

Besides, at the center section of one long-side portion (the long-side portion on the far side) of the solution containing portion 86, there is formed a catalyst containing portion 92 of circular shape extending in the depth direction. At least one-half the circumference of the catalyst containing portion 92 projects out from the solution containing portion 86. The catalyst containing portion 92 communicates with the solution containing portion 86 via a communicating window 94, which is formed at a portion that intersects the long-side portion of the solution containing portion 86, namely, the portion of one-half or less of the circumference of the catalyst containing portion 92.

The solution containing portion 86 has a pair of arcuate projecting portions 96, 96 projecting from its bottom surface 88 at its lengthwise center section (the lateral direction in FIG. 15). The pair of arcuate projecting portions 96, 96 are arranged so as to have their backs opposed to each other. The arcuate projecting portion 96 has a curvature approximately equal to the opposite ends of the oval-shaped solution containing portion 86, which has a semicircular shape, and is positioned substantially on an extended circumference of that semicircle. With this arrangement, the pair of arcuate projecting portions 96, 96 and the semicircles on the lengthwise opposite ends of the solution containing portion 86 cooperate with one another in order to form a left/right pair of lens containing portions 98, 98 in the lengthwise opposite sections of the solution containing portion 86.

Note that the each arcuate projecting portion 96 has its circumferential opposite ends separated from the peripheral wall of the solution containing portion 86, thereby providing respective communicating grooves 100 in between. Also, the projecting height of the arcuate projecting portion 96 is made smaller than the depth of the solution containing portion 86 while being approximately equal to or slightly smaller than that of the step 90.

At the lengthwise center section of the solution containing portion 86 and between the pair of the arcuate projecting portions 96, 96, there is formed a central communication area 102. The central communication area 102 is connected with the left/right pair of the lens containing portions 98, 98 via the communicating grooves 100 provided on the circumferential opposite sides of the each arcuate projecting portion 96, while being connected with the catalyst containing portion 92 via the communicating window 94 provided to the peripheral wall of the solution containing portion 86. That is, the pair of lens containing portions 98, 98 communicate with each other via the central communication area 102 while communicating with the catalyst containing portion 92.

By injecting the hydrogen peroxide solution in the solution containing portion 86, the hydrogen peroxide solution is adapted to be stored in the pair of lens containing portion 98, 98 and the catalyst containing portion 92 as well. It is desirable that the volume of the injected hydrogen peroxide solution be adjusted so that the solution surface reaches a level approximately equal to or slightly lower than the top of the arcuate projecting portion 96. In the present embodiment, the expanded opening of the solution containing portion 86 which is located above the step 90 defines a common recess 104 onto which the pair of lens containing portion 98, 98 both open.

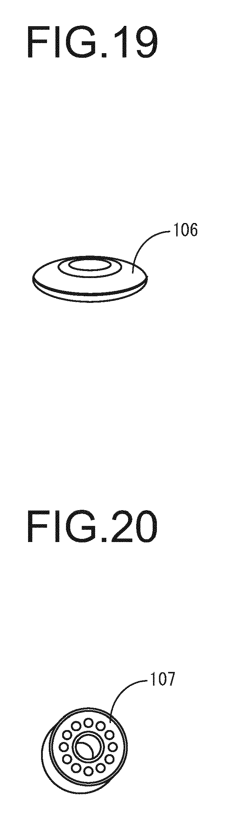

Furthermore, a metal catalyst 106 as depicted in FIG. 19 is housed within the catalyst containing portion 92. The same as the metal catalyst 14 of the first embodiment, the metal catalyst 106 may be formed of a metal catalyst made of a single material or a plurality of materials, or alternatively, a composite material in which a metal catalyst layer is adhered to the surface of a suitable base material. It would also be acceptable for the metal catalyst 106 to adjust the buoyancy by forming a void in its interior, or to adjust the surface area or the buoyancy to be exerted thereon by forming concave portions or convex portions thereto. That is, when contained in the catalyst containing portion 92 and immersed within the hydrogen peroxide solution, it will suffice for the metal catalyst 106 to have a portion that exhibits catalysis on at least a part of its surface that comes into contact with the hydrogen peroxide solution, as well as to be permitted displacement within the catalyst containing portion 92 and to displace by means of an appropriate buoyancy.

Specifically, other than the metal catalyst 106 as depicted in FIG. 19 which has a circular disk shape with the outside diameter dimension smaller than the inside diameter dimension of the catalyst containing portion 92, it is possible to employ, for example, a metal catalyst 107 as depicted in FIG. 20 which has a tire shape or the like. The outside diameter dimensions of the metal catalysts 106, 107 are made larger than the opening width of the communicating window 94 so as to be prevented from unnecessary escape from the catalyst containing portion 92. In addition, the thickness dimensions of the metal catalysts 106, 107 are made smaller than the depth of the hydrogen peroxide solution contained in the catalyst containing portion 92 so as to be permitted emerging/submerging displacement in the depth direction within the catalyst containing portion 92.

As in the first embodiment, in the case for sterilizing contact lenses constructed as above according to the present embodiment as well, hydrogen peroxide solution is injected and stored in the solution containing portion 86 of the case main unit 80, the metal catalyst 106 is input in the catalyst containing portion 92, and the contact lenses are placed in the pair of lens containing portions 98, 98, thereby subjecting the contact lenses to a sterilization process. Note that the same as in the first embodiment, it would also be possible to prepare a lid having a shape corresponding to that of the surface of the case main unit 80 so as to cover the openings of the catalyst containing portion 92 and the lens containing portions 98, 98 of the case main unit 80.

At the beginning of the sterilization process, the metal catalyst 106 emerges within the hydrogen peroxide solution based on action of its own buoyancy and the buoyancy exerted by the generated oxygen gas (the external force). On the other hand, when the lens sterilization process for a prescribed time period is completed and the hydrogen peroxide solution is neutralized, the buoyancy of the oxygen gas (external force) dissipates and the metal catalyst 106 submerges within the hydrogen peroxide solution.

It is desirable that the metal catalyst 106 partially jut out from the solution during its emergence and come into abutment with the bottom surface of the catalyst containing portion 92 during its submergence. However, the above description is not a limitation in any way. It will suffice for the metal catalyst 106 to undergo emerging/submerging displacement in the vertical direction within the solution. Besides, in the present embodiment, in order to make it easy to observe from the outside the emerging/submerging displacement of the metal catalyst 106 within the solution, it is desirable that at least the outer peripheral wall of the catalyst containing portion 92 be transparent in the case main unit 80.

In particular, with the metal catalyst having a circular disk shape as depicted in FIG. 19 or FIG. 20, it is desirable that a shaft-like portion (while not illustrated) that projects to one side along the center axis be integrally formed. By so doing, the shaft-like portion hangs downward in the solution so as to keep the upper portion of the circular disk horizontal in a substantially stable manner, and is able to prevent unstable displacement of the circular disk such as flipping over, being upright or the like.

In addition, the metal catalyst having a circular disk shape as shown in FIG. 19 or FIG. 20 is likely to undergo not only displacement of emerging or submerging but also displacement of swinging or rotation according to the position where the oxygen gas is generated due to decomposition reaction of the hydrogen peroxide solution. Owing to this swinging or rotational displacement, the hydrogen peroxide solution will even more effectively be stirred.

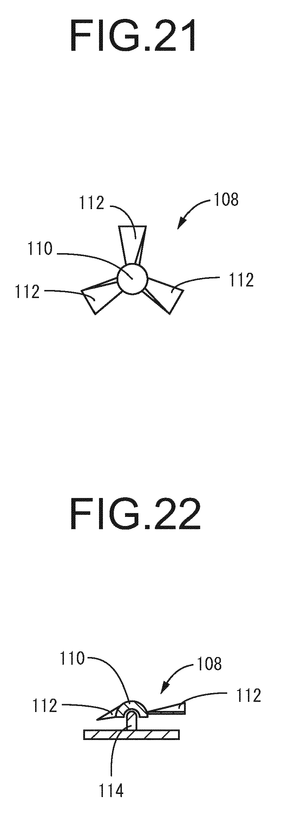

As to the case main unit 80 according to the present embodiment, it would also be possible to employ a metal catalyst 108 as shown in FIGS. 21 and 22 that rotationally displaces. The metal catalyst 108 has a shape that resembles a screw or a propeller, and includes a plurality of blades 112 that radially project from a center boss 110. The tip diameter of the blade 112 is made smaller than the inside diameter of the catalyst containing portion 92. Besides, with respect to each of the blades 112 of the metal catalyst 108, a prescribed tilt angle (the tilt angle that corresponds to an angle of a screw or a propeller) is established.

As depicted in FIG. 22, a spindle 114 that projects upward from the base wall of the catalyst containing portion 92 supports the center boss 110. With this arrangement, the metal catalyst 108 is supported so as to be rotatable about its center axis extending in the vertical direction within the catalyst containing portion 92. Note that the mass of the metal catalyst 108 according to the present embodiment is adjusted such that even with the oxygen gas exerted on the metal catalyst 108 by means of decomposition reaction of the hydrogen peroxide solution, the metal catalyst 108 submerges in opposition to the buoyancy and is kept supported by the spindle 114.

The metal catalyst 108 as described above is disposed in the hydrogen peroxide solution within the catalyst containing portion 92, thereby undergoing rotational displacement based on action of the buoyancy (the external force) exerted on the blades 112 by the oxygen gas. Thus, at the beginning of the contact lens sterilization process as mentioned above, the metal catalyst 108 rotates in a relatively forceful manner. On the other hand, when the sterilization process for a prescribed time period is completed and the hydrogen peroxide solution is neutralized, the rotational force dissipates and the metal catalyst 108 stops rotating.

Therefore, by observing the metal catalyst 108 start rotational displacement and be held in a non-displacement state, the user is able to check the status of the contact lens sterilization process, the same as the above-mentioned metal catalyst 106 that undergoes emerging/submerging displacement.

With the metal catalyst 108 of this rotation type, it is not necessary to adjust its own buoyancy with high accuracy in comparison with the case employing the metal catalyst 106 of emergence/submergence type. Accordingly, there is a greater degree of freedom in selecting the material or the like, thereby achieving an advantage of avoiding a problem of operation failure depending on accuracy of the buoyancy.

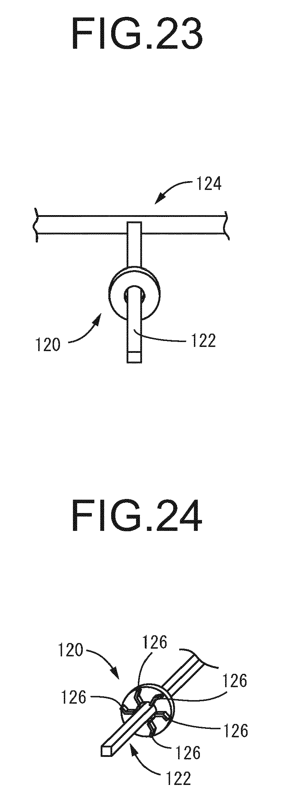

Another embodiment of the catalyst of rotation type is depicted in FIG. 23. A metal catalyst 120 according to the present embodiment has a ring shape or a cylindrical shape, and is adapted to be mounted onto a case main unit (not shown) so as to be rotatable in the circumferential direction about its center axis. In FIG. 23, 122 denotes a spindle member that is furnished with a fastening shaft 124 secured to the case main unit (not shown). The metal catalyst 120 of annular shape is externally fitted around the spindle member 122 in a rotatable manner so as to be mounted thereto. The metal catalyst 120 is mounted such that the fastening shaft 124 is inserted into and secured to, for example, the parallel communication groove 38 of the case main unit 10 according to the first embodiment, while the spindle member 122 extending horizontally within the catalyst containing portion 24. In this state, the metal catalyst 120 rotatably supported by the spindle member 122 is immersed and rotatably disposed in the hydrogen peroxide solution 44 within the catalyst containing portion 24. Of course, it would also possible for the metal catalyst 120 to establish its inside diameter dimension sufficiently greater than the outside diameter dimension of the spindle member 122 or the like so as to be permitted emerging/submerging displacement as well within the hydrogen peroxide solution.

It is desirable to provide a bubble trap to the metal catalyst 120 shown in FIG. 23 for trapping the generated oxygen bubbles and exerting the buoyancy of the trapped oxygen bubbles as a rotational force in one direction with respect to the metal catalyst 120 in order to induce its rotational displacement even more efficiently. Specifically, the bubble trap is preferably formed as a recess that opens in one circumferential side of the metal catalyst 120. For example, FIG. 24 depicts a bubble trap 126 comprising L-shaped or U-shaped protrusions that are formed on one axial end face or on both axial end faces of the metal catalyst 120 and open in one rotational direction. Alternatively, it would also be acceptable to employ a plate-shaped bubble trap that projects from the outside peripheral face of the metal catalyst 120 and tilts toward one circumferential direction. Generally, the constructions pursuant to an actuating force generating mechanism of a water wheel or the like are appropriately employable.

Note that the metal catalyst 120 of rotation type is not limited to the annular shape as depicted in FIG. 23. Various shapes such as a star, a propeller, a petal, a gear, or the like could be selected for the purpose of considering design effect, operation efficiency of the rotational force on the basis of the buoyancy of oxygen bubbles, or the like.

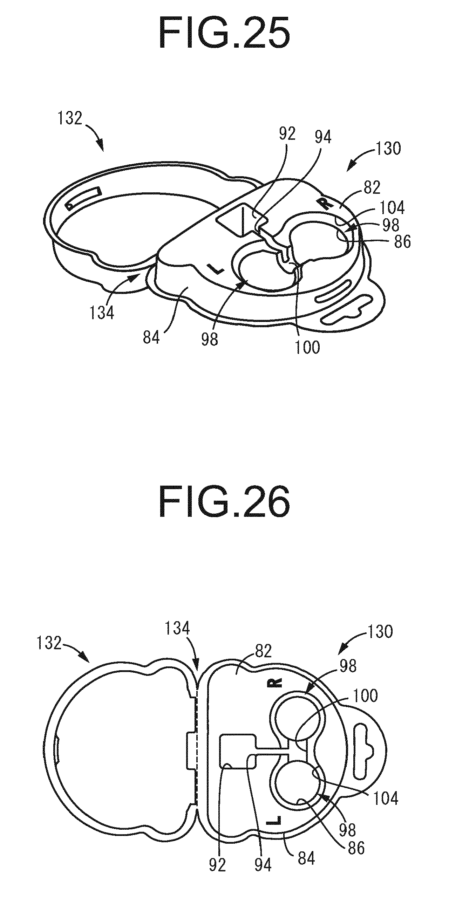

Moreover, the case main unit and the lid may be linked with each other via a bendable hinge part. In that case, it would be possible for the case main unit and the lid to be formed as separate elements and linked by a hinge or the like, or alternatively, to be integrally molded so as to be linked by a thin-walled bendable section. As a specific example, as depicted in FIGS. 25 and 26 according to a third embodiment of the present invention, by press-forming using a film material made of a synthetic resin such as polyethylene terephthalate, polypropylene, or the like, a structure integrally equipped with a case main unit 130 and a lid 132 can be provided. Specifically, the case main unit 130 and the lid 132 have a linear section extending along the outer peripheral edge thereof for a predetermined length, and are integrally linked to each other by the linear section in a bendable manner. By this linking portion 134 being bent, the lid 132 is bent and superposed against the case main unit 130. With this arrangement, the solution containing portion 86 is adapted to be covered by means of the lid 132 being superposed against the upper face of the case main unit 130 onto which the solution containing portion 86 opens. In order to bend the linking portion 134 with enhanced ease of operation, it would be effective to provide perforation-like discontinuous slits, for example. In FIGS. 25 and 26, as an aid to understanding, elements like those in the second embodiment shall be designated by like reference numerals.

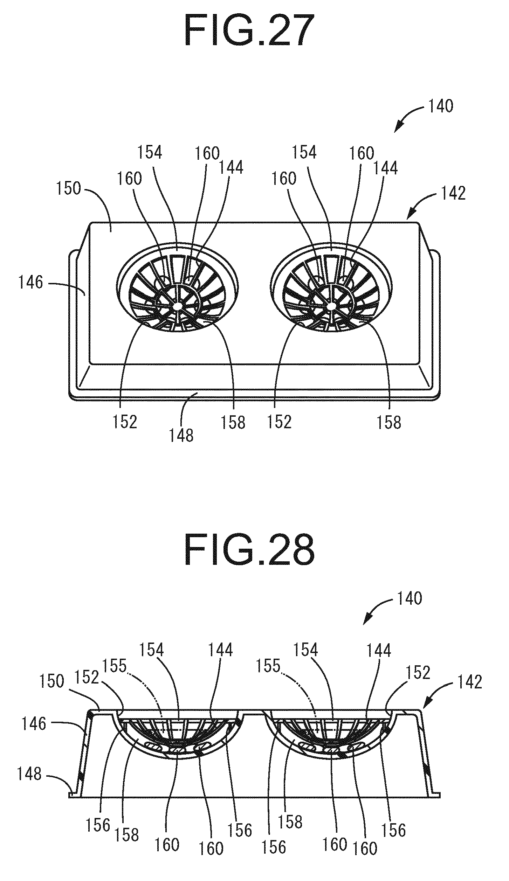

Next, FIGS. 27 and 28 depict a case for sterilizing contact lenses 140 according to a fourth embodiment of the present invention. The case for sterilizing contact lenses 140 has a structure in which a case main unit 142 of oblong, generally rectangular block shape is furnished with a left/right pair of lens containing portions 144, 144 that open onto the upper face of the case main unit 142. While not illustrated, a lid is separately provided so as to be superposed against the case main unit 142 from the above and be detachably mounted thereto. By the lid being superposed against the upper face and the outside peripheral face of the case main unit 142, the pair of lens containing portions 144, 144 will be covered.

The case main unit 142 described above has a hollow structure that opens downward, and is constituted by an integrally resin molded component having a prescribed thickness in its entirety. Further, the case main unit 142 includes a flange portion 148 integrally formed with the rim of the lower opening of a peripheral wall 146 so as to jut out to the outside peripheral face of the case main unit 142. Owing to the lower end face of the flange portion 148, the case main unit 142 can be placed and used on a generally horizontal support surface such as a table.

Moreover, the case main unit 142 has on its upper wall 150 semispherical recesses 152 that open upward respectively in left and right part. The pair of recesses 152 define the solution containing portion. Furthermore, a basket-shaped fence 154 is attached to each of the recesses 152 so as to be contained therein. The basket-shaped fence 154 is constituted by a molded component made of a synthetic resin material, for example, and has a concave structure depressing downward that resembles a shallow basket or sieve. That is, the basket-shaped fence 154 has a porous structure in which a plurality of frames are interconnected so as to form gaps among the frames. In the present embodiment in particular, frames that radially extend in the diametrical direction and frames that annularly extend in the circumferential direction are integrally connected so as to provide the porous structure. A curvature radius of the spherical concave face of the basket-shaped fence 154 is greater than the curvature radius of the convex side of a contact lens 155.

The basket-shaped fence 154 is contained in the recess 152 of the case main unit 142 so that the convex side thereof faces downward. The outer peripheral portion of the basket-shaped fence 154 is supported by the inside peripheral wall face of the recess 152 of the case main unit 142, so that the basket-shaped fence 154 is disposed so as to be supported at the middle section of the recess 152 in the depth direction. In addition, on the inside face of the recess 152 of the case main unit 142, there is formed a fence support projection 156 projecting from the middle section in the depth direction. The fence support projection 156 detains and locates the outer peripheral portion of the basket-shaped fence 154 with support, and secures it if needed, so that the basket-shaped fence 154 is fixedly attached to the prescribed location within the recess 152.

By so doing, within the recess 152 of the case main unit 142, the lens containing portion 144 is formed above the basket-shaped fence 154. The contact lens 155 is adapted to be contained in the lens containing portion 144 while being supported by the basket-shaped fence 154. That is, within the recess 152 serving as the solution containing portion, the area above the basket-shaped fence 154 defines the lens containing portion 144. With support of the basket-shaped fence 154, the contact lens 155 is subjected to a sterilization process while being immersed in the hydrogen peroxide solution contained within the recess 152.

Meanwhile, by means of the basket-shaped fence 154 being disposed at the middle section in the depth direction of the recess 152 of the case main unit 142, there is formed a catalyst containing portion 158 below the basket-shaped fence 154, at the base part of the recess 152. A metal catalyst 160 is housed within the catalyst containing portion 158. The metal catalyst 160 may be formed of a metal catalyst made of a single material or a plurality of materials, or alternatively, a composite material in which a metal catalyst layer is adhered to the surface of a suitable base material, the same as the metal catalyst 14 according to the first embodiment. In the present embodiment in particular, it is not necessary to adjust the buoyancy of the metal catalyst 160, and the metal catalyst 160 could be separated from the bottom surface of the recess 152 or the basket-shaped fence 154, or alternatively be secured to the bottom surface of the recess 152 or the basket-shaped fence 154. Also, in the present embodiment in particular, by adjusting the size and shape of the gaps of the basket-shaped fence 154, flowing of the contained hydrogen peroxide solution will be permitted, while passing of the contact lens 155 and the metal catalyst 160 will be prevented. Therefore, it is possible to employ a granular one as the metal catalyst 160, and at the same time, to prevent the metal catalyst 160 from coming into contact with the contact lens 155. That is, the basket-shaped fence 154 prevents movement of the metal catalyst out of the catalyst containing portion 158. Accordingly, the basket-shaped fence 154 defines a contact preventing member for preventing the metal catalyst 160 from coming into contact with the contact lens 155.

Consequently, with the case for sterilizing contact lenses 140 according to the present embodiment as well, the hydrogen peroxide solution is injected into the left/right pair of recesses 152, 152 respectively, and the left and right contact lenses 155, 155 are respectively input in the lens containing portions 144, 144 formed within the pair of recesses 152, 152, so that a sterilization process of the contact lenses 155, 155 by using the hydrogen peroxide solution can be performed. At that time, the pair of lens containing portions 144, 144 are both open upward onto the upper wall 150 of the case main unit 142, and the contact lenses are contained in the lens containing portion 144 so as to be supported horizontally by the concave bottom surface of the lens containing portion 144 formed by the basket-shaped fence 154. Thus, the user is able to easily place and take out the contact lens 155 with respect to the lens containing portion 144, thereby exhibiting excellent usability.

Moreover, by employing the metal catalyst 160, the desired neutralization reaction will stably be manifested, thereby stably carrying out the desired sterilization process for the contact lens 155 with high reliability. In addition, the direct contact of the contact lens 155 to the metal catalyst 160 can be completely prevented, thereby avoiding deterioration of efficiency of catalysis due to adhesion of the contact lens 155 to the catalyst surface. Besides, progress of deposition of the heavy metal to the contact lens 155 will be prevented as practicably as possible, and damage to the contact lens 155 caused by interference to the metal catalyst 160 having a rough surface will also be avoided, thereby affording enhanced ease of use.

Furthermore, the metal catalyst 160 is placed below the contact lens 155 adapted to be contained in the lens containing portion 144. With this arrangement, in association with rise of the oxygen gas generated around the metal catalyst 160, the hydrogen peroxide solution will efficiently be circulated around the contact lens 155 contained within the lens containing portion 144 above the metal catalyst 160. Additionally, it can be expected that the bubbles of the oxygen gas passing the surface of the contact lens 155 will exhibit cleaning effect by means of the contact action or convection current owing to the oxygen gas.

As needed, a surface treatment is performed to the area which comes into contact with the contact lens 155 such as the concave side surface of the basket-shaped fence 154. The surface treatment is exemplified more specifically by mirror-like finishing or the like for improving surface roughness, or, by plasma treatment or corona discharge treatment or the like for enhancing hydrophilic property or preventing sticking of the contact lens 155.

With respect to the case for sterilizing contact lenses 140 according to the present embodiment as well, it is desirable that at least one of the case main unit 142 and the lid be formed of a transparent resin so that the user can easily observe the condition of neutralization by the metal catalyst 160, the condition of sterilization by the hydrogen peroxide solution, or the like.

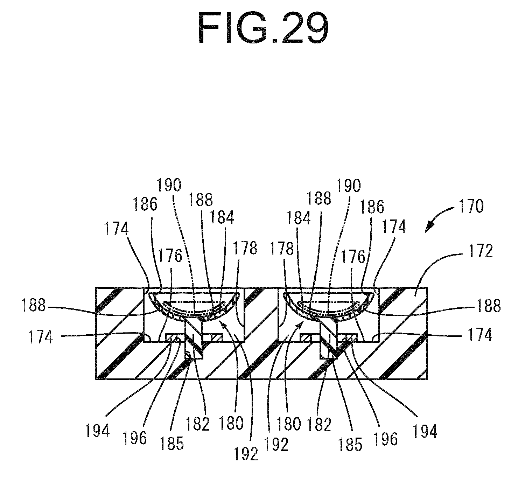

Next, FIG. 29 depicts a case for sterilizing contact lenses 170 according to a fifth embodiment of the present invention.

The case for sterilizing contact lenses 170 is furnished with a case main unit 172 of oblong, solid, and rectangular block shape. The case main unit 172 has a left/right pair of solution containing portions 174, 174 that open onto the upper face of the case main unit 172. The hydrogen peroxide solution is adapted to be contained within these solution containing portions 174, 174. Note that the same as the fourth embodiment, a lid (not shown) is adapted to detachably be mounted onto the case main unit 172 so as to cover the pair of the solution containing portions 174, 174.

The solution containing portion 174 has a circular concave shape incorporating a circular bottom surface 176 and a cylindrical inside peripheral surface 178. Inside the solution containing portion 174, there is provided a protruding support part 180 of generally umbrella or mushroom shape contained therein. The protruding support part 180 includes a leg portion 182 of straight pillar shape and an umbrella-shaped head portion 184 integrally formed with the upper end of the leg portion 182 and flaring in the axis-perpendicular direction. The lower end of the leg portion 182 is secured press-fit into a support hole 185 that opens onto the center of the bottom surface 176 of the solution containing portion 174, whereby the protruding support part 180 is formed projecting upright from the center portion of the solution containing portion 174.

The umbrella-shaped head portion 184 of the protruding support part 180 has a generally concave, semispherical shape, and to the upper end face thereof there is provided a lens containing portion 186 of generally shallow bowl shape. In addition, there are formed a plurality of through-holes 188 that pierce the umbrella-shaped head portion 184 in the thickness direction. Note that the outside diameter dimension of the umbrella-shaped head portion 184 is made slightly smaller than inside diameter dimension of the solution containing portion 174, so that there is formed an annular gap between the outside peripheral face of the umbrella-shaped head portion 184 and the inside peripheral surface 178 of the solution containing portion 174. A contact lens 190 is adapted to be placed on and contained in the lens containing portion 186 with support.

Meanwhile, in the solution containing portion 174, the area below the umbrella-shaped head portion 184, namely, the bottom-side area, defines a catalyst containing portion 192. Specifically, the solution containing portion 174 is divided by the umbrella-shaped head portion 184 into the catalyst containing portion 192 located on the bottom side and the lens containing portion 186 located on the opening side. A metal catalyst 194 is housed within the catalyst containing portion 192.

The metal catalyst 194 has an annular shape and is attached to the leg portion 182 of the protruding support part 180 in a displaceable fashion. The aperture of a center hole 196 of the metal catalyst 194 is made smaller than the outside diameter dimension of the umbrella-shaped head portion 184 of the protruding support part 180. With this arrangement, the metal catalyst 194 is prevented from becoming dislodged and detached from the protruding support part 180 by the umbrella-shaped head portion 184. As will be understood from the above description, the umbrella-shaped head portion 184 of the protruding support part 180 provides a contact preventing member for limiting movement of the metal catalyst 194 within the solution containing portion 174 as well as preventing the metal catalyst 194 from coming into contact with the contact lens 190 contained in the lens containing portion 186.

Note that the metal catalyst 194 may be formed of a metal catalyst made of a single material or a plurality of materials, or alternatively, a composite material in which a metal catalyst layer is adhered to the surface of a suitable base material. The metal catalyst 194 may also have a hollow structure. Besides, the buoyancy of the metal catalyst 194 is adjusted by: forming a hollow that opens onto its lower face and a discharge passage of the oxygen gas stored in the hollow, like the metal catalyst 14 according to the first embodiment; forming irregularities on its surface like the metal catalyst 107 depicted in FIG. 20; forming blades on its outside peripheral face like the metal catalyst 108 depicted in FIG. 21; forming a bubble trap on its side peripheral face that opens downward like the metal catalyst 120 depicted in FIG. 24; or the like.

With this arrangement, during the neutralization reaction with respect to the hydrogen peroxide solution within the catalyst containing portion 192, the metal catalyst 194 emerges on the basis of buoyancy action of the generated oxygen gas and displaces axially upward along the leg portion 182 of the protruding support part 180. Also, owing to the buoyancy of the oxygen gas exerted on the irregularities or the like formed on the surface of the metal catalyst 194, it is possible to subject the metal catalyst 194 to rotational displacement around the center axis.

On the other hand, when the neutralization reaction with respect to the hydrogen peroxide solution is completed, the buoyancy action of the oxygen gas against the metal catalyst 194 dissipates, and the metal catalyst 194 having greater specific gravity than the hydrogen peroxide solution (the water) undergoes submerging displacement. Consequently, when there is no generation of the oxygen gas, as depicted in FIG. 29, the metal catalyst 194 becomes positioned resting on the bottom surface 176 of the solution containing portion 174.

Accordingly, the same as the fourth embodiment, in the case for sterilizing contact lenses 170, the left and right contact lenses 190, 190 are horizontally supported by the respective concave bottom surfaces of the pair of lens containing portion 186, 186 so as to be contained therein. Thus, the user is able to easily place and take out the contact lens 190 with respect to the lens containing portion 186 and carry out the operation for sterilization of the contact lens 190 with ease and rapidity. Moreover, by positioning the metal catalyst in a state of non-contact with the contact lens 190, the sterilization process of the contact lens 190 can be stably performed with high reliability. Concomitantly, progress of deposition of the heavy metal to the contact lens 190 or damage to the surface of the contact lens will be effectively avoided.

In particular, in the case for sterilizing contact lenses 170 according to the present embodiment, the same as in the fourth embodiment, the metal catalyst 194 is placed only below the lens containing portion 186. This structure is different from the conventional structures as disclosed in aforementioned Patent Document 5, for example, wherein the metal catalyst is disposed over the entire inside face of the lens containing portion 186 including its side peripheral face. Therefore, the oxygen gas generated by the neutralization reaction will rise from below the contact lens 190 disposed at generally center of the lens containing portion 186 and only at the center of the lens containing portion 186, within the hydrogen peroxide solution. As a result, within the hydrogen peroxide solution contained in the lens containing portion 186, there will be effectively produced circulating flow which ascends at the center of the lens containing portion 186 while descending at the outer peripheral portion thereof. Owing to this circulating flow, the hydrogen peroxide solution within the lens containing portion 186 will be effectively stirred. Thus, the neutralization reaction will progress substantially uniformly over the entire hydrogen peroxide solution. The sterilization process of the contact lens 190 will also be performed even more stably over its entirety.

In the present embodiment, it is desirable that at least one of the case main unit 172 on the one hand, and the umbrella-shaped head portion 184 and the lid on the other, be made of a transparent resin. This arrangement makes it easy to visually observe the metal catalyst 194 from the outside at the time of the contact lens sterilization process by using the hydrogen peroxide solution, and to check the condition of the progress of the neutralization reaction in association with the sterilization process.

While the present invention has been described in detail hereinabove in terms of the preferred embodiments, the invention is not limited by the specific disclosures thereof. For example, as to the case for sterilizing contact lenses 140 depicted in FIGS. 27 and 28 or the case for sterilizing contact lenses 170 depicted in FIG. 29 as well, a communication passage for interconnecting the left/right pair of the solution containing portions may be provided, with a tunnel structure, a groove structure that opens onto the upper face, or the like. It would also be acceptable to further provide the catalyst containing portion 24 as shown in the case for sterilizing contact lenses 16 depicted in FIG. 13 for communicating with the left/right pair of the solution containing portions. With this arrangement, it is possible to supplementally locate another metal catalyst in the catalyst containing portion in addition to the metal catalyst contained in the pair of solution containing portions.

Furthermore, in the present invention, the metal catalyst needs not necessarily be movable. For example, in the case for sterilizing contact lenses 140 as depicted in FIGS. 27 and 28, it could also be possible for the metal catalyst to be secured to or formed so as to cover the surface of the center of the base part of the lens containing portion 144 that is covered by the basket-shaped fence 154. Alternatively, the metal catalyst may be formed so as to cover only the convex side surface of the basket-shaped fence 154 which does not come into contact with the contact lens 155 (the face opposed to the bottom surface of the lens containing portion 144). Besides, in the case for sterilizing contact lenses 170 as depicted in FIG. 29, it would also be acceptable for the metal catalyst to be secured to the leg portion 182 of the protruding support part 180, or to be formed so as to cover the surface of the leg portion 182.

### KEY TO SYMBOLS

10, 80, 130, 142, 172: case main unit, 12, 132: lid, 14, 106, 107, 108, 120, 160, 194: metal catalyst, 16, 140, 170: case for sterilizing contact lenses, 24, 92, 158, 192: catalyst containing portion, 26, 98, 144, 186: lens containing portion, 32: dividing wall, 44: hydrogen peroxide solution, 60: support rod, 62: retaining projection, 64: base material, 66: catalyst layer, 68: hollow, 70: discharge passage, 155, 190: contact lens

## Claims

1. A case for sterilizing contact lenses (16, 80, 130, 140, 170) comprising:
a pair of lens containing portions (26, 98, 144, 186) each opening upward for immersing and sterilizing a contact lens (155, 190) for a left eye and a contact lens (155, 190) for a right eye within a hydrogen peroxide solution (44), a bottom surface of each of the lens containing portions (26, 98, 144, 186) being adapted to support the contact lens (155, 190) horizontally in a state where either one of a concave side lens surface and a convex side lens surface faces toward an opening of the lens containing portion (26, 98, 144, 186);
a metal catalyst (14, 106, 107, 108, 120, 160, 194) which catalyzes decomposition reaction of the hydrogen peroxide solution (44), being provided at a location where the metal catalyst (14, 106, 107, 108, 120, 160, 194) comes into contact with the hydrogen peroxide solution (44); and
a contact preventing member (32, 34, 92, 94, 100, 154, 184) provided between the metal catalyst (14, 106, 107, 108, 120, 160, 194) and the lens containing portion (26, 98, 144, 186) for preventing the contact lens (155, 190) from coming into contact with the metal catalyst (14, 106, 107, 108, 120, 160, 194).

2. The case for sterilizing contact lenses (140, 170) according to claim 1, wherein the metal catalyst (160, 194) is positioned below the contact lens (155, 190) adapted to be contained in the lens containing portion (144, 186).

3. The case for sterilizing contact lenses (170) according to claim 1 or 2, further comprising: a solution containing portion (174) for containing the hydrogen peroxide solution (44); and a protruding support part (180) projecting from a bottom surface (176) of the solution containing portion (174), wherein the lens containing portion (186) is formed at a projecting distal end of the protruding support part (180).

4. The case for sterilizing contact lenses (170) according to claim 3, wherein the protruding support part (180) includes a leg portion (182) projecting from the bottom surface (176) of the solution containing portion (174) and an umbrella-shaped head portion (184) flaring peripherally outward from a projecting distal end of the leg portion (182), and the lens containing portion (186) is formed at the umbrella-shaped head portion (184).

5. The case for sterilizing contact lenses (170) according to claim 4, wherein in the solution containing portion (174), the metal catalyst (194) is positioned below the umbrella-shaped head portion (184), the contact preventing member (184) comprises the umbrella-shaped head portion (184) of the protruding support part (180), and the umbrella-shaped head portion (184) limits movement of the metal catalyst (194) within the solution containing portion (174) so as to prevent the metal catalyst (194) from coming into contact with the contact lens (190) adapted to be contained in the lens containing portion (186) of the umbrella-shaped head portion (184).

6. The case for sterilizing contact lenses (16, 80, 130, 140, 170) according to any one of claims 1-5, wherein, as the contact preventing member (32, 34, 92, 94, 100, 154, 184), a passage constricting member (32, 34, 100, 154, 184) is provided between areas where the contact lens (155, 190) and the metal catalyst (14, 106, 107, 108, 120, 160, 194) are positioned in order to prevent movement of both the contact lens (155, 190) and the metal catalyst (14, 106, 107, 108, 120, 160, 194) while permitting flow of the hydrogen peroxide solution (44).

7. The case for sterilizing contact lenses (16) according to any one of claims 1-6, further comprising a temporary lens platform (54) for temporarily retaining the contact lens, the temporary lens platform (54) being provided at a surface away from the solution containing portion (24, 26) which is adapted to contain the hydrogen peroxide solution (44).

8. The case for sterilizing contact lenses (16) according to any one of claims 1-7, wherein: the solution containing portion (24, 26) which is adapted to contain the hydrogen peroxide solution (44) includes the lens containing portion (26); a lid (12) is provided for covering the solution containing portion (24, 26); and a gas discharge passage is provided for discharging an oxygen gas, which is generated in association with a decomposition of the hydrogen peroxide solution (44), from the solution containing portion (24, 26) with the solution containing portion (24, 26) covered by the lid (12).

9. The case for sterilizing contact lenses (130) according to claim 8, wherein the lid (132) is provided in a bendable manner to a case main unit (130) which forms the solution containing portion (86), and the solution containing portion (86) is adapted to be covered by means of the lid (132) being bent with respect to the case main unit (130) so as to be superposed against an upper face of the case main unit (130) onto which the solution containing portion (86) opens.

10. The case for sterilizing contact lenses (130) according to claim 9, wherein the case main unit (130) and the lid (132) is integrally molded from a thin-walled resin material, and the lid (132) is bendable by being bent at a linking portion (134) of the case main unit (130) and the lid (132).

11. The case for sterilizing contact lenses (16, 80, 130, 170) according to any one of claims 1-10, further comprising a catalyst containing portion (24, 92, 192) which contains the metal catalyst (14, 106, 107, 108, 120, 194), wherein the pair of lens containing portions (26, 98, 186) and the catalyst containing portion (24, 92, 192) are respectively formed as recesses that open upward, and mutual communication passages (34, 100, 102, 188) are formed for mutually connecting the recesses so as to allow the hydrogen peroxide solution (44) to flow among the recesses.

12. The case for sterilizing contact lenses (16) according to claim 11, wherein each of the pair of lens containing portions (26) has an aperture of the mutual communication passage (34), and the aperture has a length equal to 1/5 or more of a circumference of the lens containing portion (26).

13. The case for sterilizing contact lenses (16) according to claim 11 or 12, wherein the mutual communication passages (34) interconnect the recesses so as to form an annular circulation passage.

14. The case for sterilizing contact lenses (16) according to claim 13, wherein:
the catalyst containing portion (24) is positioned between the pair of lens containing portions (26);
a pair of adjacent communication passages (34) each connecting with one of the lens containing portions (26) are formed on a rim of an opening of the catalyst containing portion (24) at positions opposed to each other so that the pair of lens containing portions (26) are interconnected via the catalyst containing portion (24); and
a parallel communication passage (38) is formed so as to extend parallel to a direction of array of the pair of lens containing portions (26) and the catalyst containing portion (24), and lengthwise opposite ends (40) of the parallel communication passage (38) each connect with one of the lens containing portions (26) while a lengthwise middle portion (42) of the parallel communication passage (38) connects with the catalyst containing portion (24) so that the circulation passage includes the pair of adjacent communication passages (34) and the parallel communication passage (38).

15. The case for sterilizing contact lenses (16, 80, 130) according to any one of claims 11-14, further comprising a common recess (36, 104) that surrounds a periphery of openings of the pair of lens containing portions (26, 98) and the catalyst containing portion (24, 92), wherein the pair of lens containing portions (26, 98) and the catalyst containing portion (24, 92) open onto the common recess (36, 104).

16. The case for sterilizing contact lenses (16) according to any one of claims 11-15, wherein the lid (12) that covers the opening of the pair of lens containing portions (26) has on an inside face thereof inward convex portions (54) corresponding with the lens containing portions (26), and by means of the lid (12) being mounted onto the opening of the lens containing portions (26), the inward convex portions (54) are adapted to be inserted into the hydrogen peroxide solution (44) contained in the lens containing portions (26).

17. The case for sterilizing contact lenses (16) according to claim 16, wherein by means of the lid (12) being mounted onto the opening of the lens containing portions (26), the inward convex portions (54) are adapted to push the contact lenses into the hydrogen peroxide solution (44) contained in the lens containing portions (26).

18. The case for sterilizing contact lenses (16, 80, 130, 140, 170) according to any one of claims 1-17, wherein the metal catalyst (14, 106, 107, 108, 120, 160, 194) is at least one of metals and metal oxides thereof selected from the group consisting of platinum, silver, palladium, copper, manganese, cobalt, and aluminum.

19. The case for sterilizing contact lenses (16, 80, 130, 140, 170) according to any one of claims 1-18, wherein the metal catalyst (14, 106, 107, 108, 120, 160, 194) has a surface area of between 3 and 30 cm² per 10 ml of the hydrogen peroxide solution (44).

20. The case for sterilizing contact lenses (16, 80, 130, 140, 170) according to any one of claims 1-19, wherein the metal catalyst (14, 106, 107, 108, 120, 160, 194) is made by adhering a metal coat (66) which catalyzes decomposition reaction of the hydrogen peroxide solution (44) to a surface of a base material (64).

21. The case for sterilizing contact lenses (16, 80, 130, 170) according to any one of claims 1-20, wherein:
the metal catalyst (14, 106, 107, 108, 120, 194) is disposed in the catalyst containing portion (24, 92, 192) and adapted to be immersed within the hydrogen peroxide solution (44);
during decomposition reaction of the hydrogen peroxide solution (44), the metal catalyst (14, 106, 107, 108, 120, 194) undergoes displacement by exertion of buoyancy on the basis of oxygen bubbles being generated; and
after decomposition reaction of the hydrogen peroxide solution (44), the metal catalyst (14, 106, 107, 108, 120, 194) is held in non-displacement state due to dissipation of the buoyancy on the basis of the oxygen bubbles while being displaceable within the catalyst containing portion (24, 92, 192).

22. The case for sterilizing contact lenses (16, 80, 130, 170) according to claim 21, wherein the displacement of the metal catalyst (14, 106, 107, 108, 120, 194) during decomposition reaction of the hydrogen peroxide solution (44) is rotation; and the non-displacement state of the metal catalyst (14, 106, 107, 108, 120, 194) after decomposition reaction of the hydrogen peroxide solution (44) is stop of rotation.

23. The case for sterilizing contact lenses (16, 80, 130, 170) according to claim 21 or 22, wherein the displacement of the metal catalyst (14, 106, 107, 108, 120, 194) during decomposition reaction of the hydrogen peroxide solution (44) is emergence; and the non-displacement state of the metal catalyst (14, 106, 107, 108, 120, 194) after decomposition reaction of the hydrogen peroxide solution (44) is submergence.

24. The case for sterilizing contact lenses (16, 80, 130, 170) according to any one of claims 21-23, wherein the metal catalyst (14, 106, 107, 108, 120, 194) has on a surface thereof at least one of a concave portion (68) and a convex portion.

25. The case for sterilizing contact lenses (16, 170) according to claim 24, wherein the metal catalyst (14, 194) includes on the surface thereof a hollow (68) serving as the concave portion (68) that opens downward with the metal catalyst (14, 194) immersed within the hydrogen peroxide solution (44), and the metal catalyst (14, 194) further includes a discharge passage (70) for discharging the oxygen bubbles trapped in the hollow (68) in a limiting manner.

26. The case for sterilizing contact lenses (16, 80, 130, 170) according to any one of claims 21-25, wherein with respect to the metal catalyst (14, 106, 107, 108, 120, 194), a ratio M/F between a mass M and a buoyancy F exerted within the hydrogen peroxide solution (44) is more than 1 and does not exceed 2.

27. The case for sterilizing contact lenses (16, 170) according to any one of claims 21-26, further comprising a reserving member (60, 62, 184, 196) that permits displacement of the metal catalyst (14, 194) within the catalyst containing portion (24, 192) in emerging and submerging directions while reserving the metal catalyst (14, 194) within the catalyst containing portion (24, 192) during discharge of the hydrogen peroxide solution (44) from the catalyst containing portion (24, 192).

28. The case for sterilizing contact lenses (16, 170) according to claim 27, wherein the metal catalyst (14, 194) includes a mating portion (60, 196), and on the basis of mating action of the mating portion (60, 196), displacement of emergence and submergence of the metal catalyst (14, 194) within the catalyst containing portion (24, 192) is permitted while detachment of the metal catalyst (14, 194) from the catalyst containing portion (24, 192) is prevented so as to provide the reserving member (60, 62, 184, 196).

29. The case for sterilizing contact lenses (16) according to claim 28, wherein the case for sterilizing contact lenses (16) as defined in claim 14 is employed, and the mating portion (60) provided to the metal catalyst (14) projects out from the catalyst containing portion (24) so as to be mated with the parallel communication passage (38).
